(19) Europäisches Patentamt
European Patent Office
Office européen des brevets

(11) Veröffentlichungsnummer: **0 054 677**
**B1**

(12) # EUROPÄISCHE PATENTSCHRIFT

(45) Veröffentlichungstag der Patentschrift: **29.05.85**

(51) Int. Cl.⁴: **C 07 D 501/36,** A 61 K 31/545

(21) Anmeldenummer: **81108768.3**

(22) Anmeldetag: **23.10.81**

(54) **Neue Cephalosporine, Verfahren zu ihrer Herstellung und diese Verbindungen enthaltende Arzneimittel.**

(30) Priorität: **13.12.80 DE 3047082**

(43) Veröffentlichungstag der Anmeldung: **30.06.82 Patentblatt 82/26**

(45) Bekanntmachung des Hinweises auf die Patenterteilung: **29.05.85 Patentblatt 85/22**

(84) Benannte Vertragsstaaten: **AT BE CH DE FR IT LI LU NL SE**

(56) Entgegenhaltungen:
**EP - A - 0 021 176**
**EP - A - 0 022 494**
**EP - A - 0 035 161**

(73) Patentinhaber: **Dr. Karl Thomae GmbH, Postfach 1755, D-7950 Biberach (Riss) (DE)**

(72) Erfinder: **Wetzel, Bernd, Dr. Dipl.-Chem., Kapellenweg 21, D-7950 Biberach 1 (DE)**
Erfinder: **Woitun, Eberhard, Dr. Dipl.-Chem., Stecherweg 17, D-7950 Biberach 1 (DE)**
Erfinder: **Reuter, Wolfgang, Dr. Dipl.-Chem., Nelkenweg 10, D-7951 Laupertshausen (DE)**
Erfinder: **Maier, Roland, Dr. Dipl.-Chem., Bodelschwinghstrasse 39, D-7950 Biberach 1 (DE)**
Erfinder: **Lechner, Uwe, Dr., Panoramastrasse 12, D-7951 Ummendorf (DE)**
Erfinder: **Goeth, Hanns, Dr., Oberer Bühl 6, D-7950 Biberach 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 054 677 B1

BUNDESDRUCKEREI BERLIN

**Beschreibung**

Die Erfindung betrifft neue Cephalosporine der allgemeinen Formel I

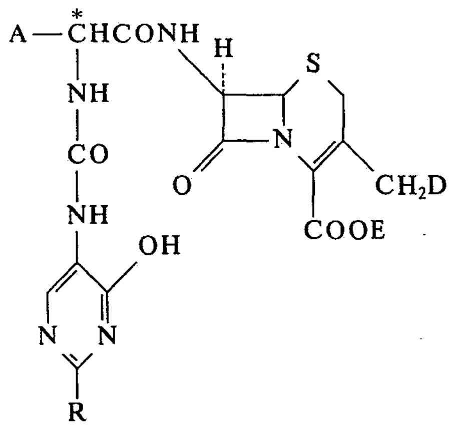

(I)

bzw. deren mögliche Tautomere, ihre physiologisch verträglichen Salze mit anorganischen oder organischen Basen, Verfahren zur Herstellung dieser Verbindungen und diese Verbindungen enthaltende Arzneimittel.

In der allgemeinen Formel I bedeuten:

A die Phenyl-, 4-Hydroxyphenyl-, 3,4-Dihydroxyphenyl-, oder die 2- oder 3-Thienylgruppe,

D die Gruppe SHet, wobei Het die 4H-5,6-Dioxo-1,2,4-triazin-3-yl- oder 4-Methyl-5,6-dioxo-1,2,4-triazin-3-ylgruppe, die 1-Vinyl-tetrazol-5-yl- oder 1-Allyltetrazol-5-ylgruppe oder eine Gruppe der allgemeinen Formel II,

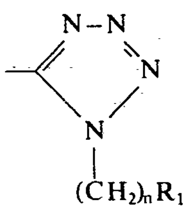

(II)

bedeutet,

in der n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure oder Sulfonsäuregruppe steht, oder in der $(CH_2)_nR_1$ eine Alkylgruppe mit 2 bis 4 Kohlenstoffatomen oder einen 2,3-Dihydroxypropylrest bedeutet,

R eine Gruppe der allgemeinen Formel $NHR_2$, in der $R_2$ eine substituierte oder unsubstituierte 3-Pyridyl-, 5-Pyrimidinyl-, 2-Thienyl-, 2-Furylmethyl-, 2-Thienylmethyl-, 2-Imidazolylmethyl-, 2-Thiazolylmethyl-, 3-Pyridylmethyl- oder eine 5-Pyrimidinylmethylgruppe bedeutet, wobei diese Gruppen durch ein Chloratom, eine Methyl-, Aacetylamino-, Hydroxy-, Methylsulfinyl-, Methylsulfonyl-, Aminocarbonyl- oder Aminosulfonylgruppe substituiert sein können,

E ein Wasserstoffatom oder eine in vitro oder in vivo leicht spaltbare Carboxylschutzgruppe. Als Carboxylschutzgruppen kommen im Sinne der Erfindung solche in Frage, welche auch bisher schon auf dem Gebiet der Penicilline und Cephalosporine eingesetzt wurden, insbesondere esterbildende Gruppen, die durch Hydrogenolyse oder Hydrolyse oder andere Behandlungen unter milden Bedingungen entfernt werden können oder esterbildende Gruppen, welche leicht im lebenden Organismus abgespalten werden können.

Beispiele für in vitro leicht spaltbare Schutzgruppen sind z. B. die Benzyl-, Diphenylmethyl-, Trityl-, t-Butyl-, die 2,2,2-Trichloräthyl- oder die Trimethylsilylgruppe. Beispiele für in vivo leicht spaltbare Schutzgruppen sind z. B. Alkanoyloxyalkylgruppen, wie z. B. die Acetoxymethyl-, Propionyloxymethyl-, 2-Acetoxy-äthyl- oder Pivaloyloxymethylgruppe oder die Phthalidylgruppe. Bedeutet E ein Wasserstoffatom, so fallen unter den Anspruch auch pharmakologisch verträgliche Salze mit anorganischen oder organischen Basen, wie z. B. die Alkali- oder Erdalkalisalze, z. B. die Natrium-, Kalium-, Magnesium- oder Calciumsalze, die Ammoniumsalze oder organische Aminsalze, z. B. solche mit Triäthylamin oder Dicyclohexylamin.

Das Sternchen an dem Kohlenstoffatom in den Verbindungen der allgemeinen Formel I bedeutet ein

Asymmetriezentrum.

Die Cephalosporinverbindungen der allgemeinen Formel I und die nachstrehend beschriebenen Zwischenprodukte können in 2 tautomeren Formen bezüglich des Pyrimidinrings vorliegen. Es hängt besonders vom jeweiligen Lösungsmittel und von der Art des Substituenten R ab, welche der nachstehenden Formen I oder I' überwiegt:

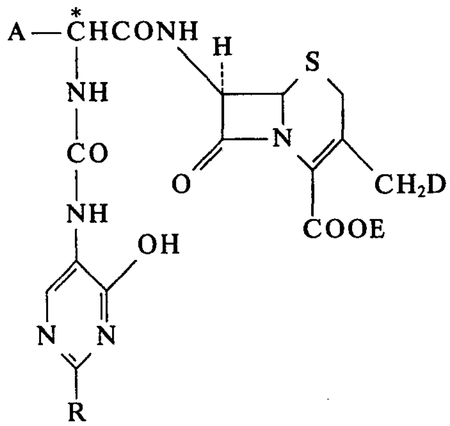

(I)

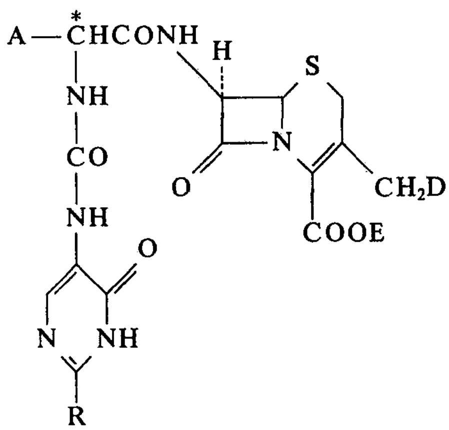

(I')

Es versteht sich von selbst, daß die eingangs angegebenen Verbindungen der allgemeinen Formel I immer beide Tautomeren umfassen.

Die Verbindungen der allgemeinen Formel I können bezüglich des mit einem Sternchen gekennzeichneten Chiralitätszentrums C+ in den beiden möglichen R- und S-Konfigurationen, jedoch auch als ein Gemisch dieser Konfigurationen vorliegen.

Die Verbindungen der allgemeinen Formel I können wie folgt hergestellt werden:

1. Durch Umsetzung eines 7-Aminocephalosporansäurederivates der allgemeinen Formel III,

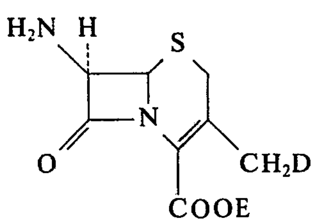

(III)

in der D die Gruppe –SHet bedeutet und E die oben genannten Bedeutungen hat, mit Ureidocarbonsäuren der allgemeinen Formel IV,

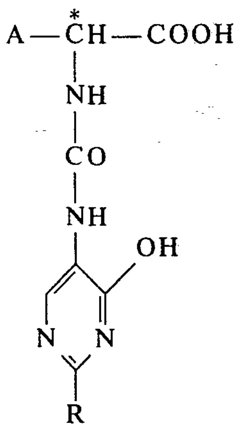

(IV)

in der A und R die vorstehende Bedeutung haben, oder ihren Salzen oder reaktiven Derivaten. Als reaktive Derivate der Ureidocarbonsäuren der allgemeinen Formel IV kommen beispielsweise deren Säureanhydride, wie z. B. die, die sich von Chlorameisensäureestern, z. B. Chlorameisensäureäthyl- oder -isobutylester, ableiten, oder deren reaktive Ester, wie der p-Nitrophenylester oder der N-Hydroxysuccinimidester, oder deren reaktive Amide, wie das N-Carbonylimidazol, aber auch deren Säurehalogenide, wie das entsprechende Säurechlorid oder deren Säureazide in Frage. Prinzipiell können jedoch alle Verknüpfungsmethoden, wie sie aus der $\beta$-Lactamchemie bekannt sind, verwendet werden.

Die Ureidocarbonsäure, ihre Salze oder ihre reaktiven Derivate werden mit den 7-Aminocephalosporansäurederivaten in einem Lösungsmittel bei Temperaturen zwischen −40°C und +40°C gegebenenfalls in Gegenwart einer Base, umgesetzt. Wird z. B. ein Anhydrid der Ureidocarbonsäure, beispielsweise das Anhydrid mit dem Äthylchloroformiat, eingesetzt, so wird die Reaktion unter Kühlung, beispielsweise bei −10°C bis +120°C in einem Lösungsmittel wie Aceton, Tetrahydrofuran, Dimethylformamid, Chloroform, Dichlormethan, Hexametapol oder in einem Gemisch dieser Lösungsmsittel, durchgeführt. Setzt man beispielsweise einen N-Hydroxysuccinimidester der Ureidocarbonsäure mit Derivaten der allgemeinen Formel III um, so wird die Reaktion vorzugsweise bei 0 bis 20°C in Anwesenheit einer Base, wie z. B. Triäthylamin, in einem Lösungsmittel wie Dimethylformamid, Dichlormethan, Dioxan oder in einem Gemisch solcher Lösungsmittel durchgeführt.

Die Umsetzung einer Ureidocarbonsäure der allgemeinen Formel IV selbst oder ihrer Salze mit Verbindungen der allgemeinen Formel III erfolgt vorteilhafterweise in Gegenwart eines Kondensationsmittels, z. B. in Gegenwart von N,N'-Dicyclohexylcarbodiimid.

2. Durch Umsetzung von Cephalosporansäurederivaten der allgemeinen Formel V oder ihrer Salze

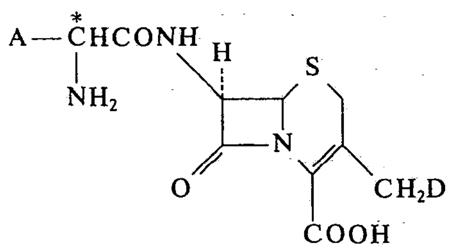

(V)

mit einem Pyrimidinderivat der allgemeinen Formel VI,

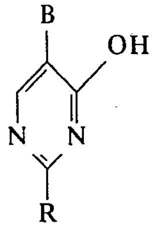

(VI)

in der R wie oben definiert ist und B die Gruppe −NCO oder ein reaktives Derivat der Gruppe −NHCOOH bedeutet, wie z. B. die Gruppen −NHCOCl, −NHCOBr oder

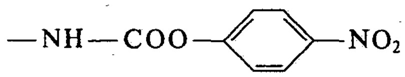

wobei die Gruppe NHCOCl besonders bevorzugt ist.
Es können auch Gemisch von solchen Pyrimidinderivaten der allgemeinen Formel VI verwendet werden, in der B teils die eine und teils die andere der vorstehend genannten Bedeutungen besitzt, z. B. die Gruppen

—NCO und 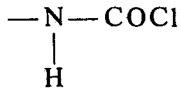

gleichzeitig nebeneinander.
Die Reaktion wird bevorzugt in beliebigen Mischungen von Wasser mit solchen organischen Lösungsmitteln, die mit Wasser mischbar sind, wie Ketonen, z. B. Aceton, cyclische Äther, z. B. Tetrahydrofuran oder Dioxan, Nitrilen, z. B. Acetonitril, Formamiden, z. B. Dimethylformamid, Dimethylsilfoxid oder Alkoholen, z. B. Isopropanol oder in Hexametapol durchgeführt. Besonders bevorzugt ist ein Gemisch aus Tetrahydrofuran und Wasser. Dabei hält man den pH der Reaktionsmischung durch Zusatz von Basen oder Verwendung von Pufferlösungen in einem pH-Bereich von etwa 2,0 bis 9,0, vorzugsweise zwischen pH 6,5 und 8,0.

3. Zur Herstellung von Verbindungen der allgemeinen Formel I bzw. I', in der D für —SHet steht, durch Umsetzung einer Verbindung der allgemeinen Formel VII,

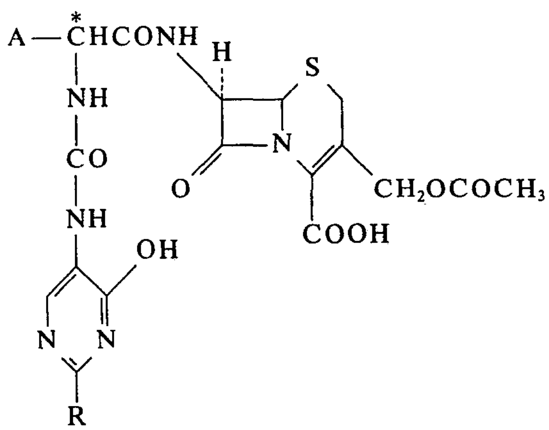

(VII)

in der A und R die oben angegebenen Bedeutungen haben mit einer Verbindung der allgemeinen Formel VIII,

Het—S—M (VIII)

in der Het die oben angegebenen Bedeutungen hat und M für ein Wasserstoffatom oder ein Alkalimetall oder ein Erdalkalimetall steht. Dazu wird z. B. eine Verbindung der Formel VII mit beispielsweise 5-Vinyl-2-mercapto-1,2,3,4-tetrazol in einem Lösungsmittel, z. B. Wasser, Methanol, Äthanol, Aceton, Methyläthylketon, Tetrahydrofuran, Acetonitril, Essigsäureäthylester, Dimethoxyäthan, Dimethylformamid, Dimethylsulfoxid, Chloroform oder einem Gemisch dieser Lösungsmittel umgesetzt. Vorzugsweise wird ein stark polares Lösungsmittel wie Wasser verwendet: In diesem Fall wird der pH-Wert der Reaktionslösung vorteilhafterweise auf 2—10 und insbesondere auf 4—8 gehalten. Der gewünschte pH-Wert kann durch Zugabe einer Pufferlösung, wie Natriumphosphat, eingestellt werden. Die Reaktionsbedingungen unterliegen keinen besonderen Beschränkungen. Normalerweise wird die Umsetzung bei einer Temperatur im Bereich von 0° bis 100°C während einer Zeitdauer von einigen Stunden durchgeführt.

Die nach den vorstehenden Verfahren hergestellten Verbindungen der allgemeinen Formel I, in der E einen anderen Rest als ein Wasserstoffatom bedeutet, können in an sich bekannter Weise zur Abspaltung der Schutzgruppe behandelt werden. Es werden dadurch die Verbindungen erhalten, in denen E Wasserstoff bedeutet und die im Sinne der Erfindung als ganz besonders bevorzugte Endverbindungen gelten. Beispielsweise wird eine Verbindung der allgemeinen Formel I, in der E für eine Diphenylmethylgruppe steht, in bekannter Weise mit Anisol und Trifluoressigsäure zur Abspaltung der Esterschutzgruppe behandelt oder es kann in ebenfalls bekannter Weise eine Silylschutzgruppe durch wäßrige Hydrolyse entfernt werden.

Die Verbindungen der allgemeinen Formel I, in der E ein Natrium- oder Kaliumkation darstellt, werden durch Umsetzung der entsprechenden freien Säure der Verbindungen der allgemeinen Formel I, in der E ein Wasserstoffatom darstellt, mit dem entsprechenden salzbildenden Ion hergestellt.

Hierzu eignet sich zum Beispiel die in der Chemie der Penicilline und Cephalosporine übliche Umsetzung mit Natriumäthylhexanoat, oder die Umsetzung mit Natriumhydrogencarbonat und anschließende Gefriertrocknung. Die Cephalosporinantibiotika der allgemeinen Formel I, in der E ein Wasserstoffatom bedeutet, können auf bekannte Weise in die Acyloxyalkylester, worin E z. B. eine Pivaloyloxymethyl rest

$$-CH_2-O\underset{\underset{O}{\|}}{C}-C(CH_3)_3$$

darstellt, überführt werden, indem man ein Alkalisalz der Cephalosporincarbonsäure, beispielsweise ein Natrium- oder Kaliumsalz, mit einem Pivaloyloxymethylhalogenid der Formel

$$Hal-CH_2-O-\underset{\underset{O}{\|}}{C}-C(CH_3)_3$$

worin Hal für Chlor, Brom oder Jod steht, umsetzt. Weitere geeignete Acyloxyalkylhalogenide sind z. B. Chlormethylacetat, Brommethylpropionat oder 1-Bromäthylacetat.

Bei Verwendung der entsprechenden Ausgangsverbindungen ist es möglich, die Verbindungen der allgemeinen Formel I in Form der Racemate oder in Form der einzelnen Isomeren herzustellen. Wenn das Endprodukt in der D,L-Form anfällt, gelingt es, die reinen D- und L-Diastereoisomeren durch präparative Flüssigkeitschromatographie (HPLC) herzustellen. Die Erfindung betrifft die Racemate und die Isomeren.

Die Ureidocarbonsäuren der allgemeinen Formel IV, die Pyrimidine der allgemeinen Formel VI, sowie die Cephalosporine der allgemeinen Formel VII sind literaturbekannt. Sie werden in der deutschen Offenlegungsschrift 29 24 296 beschrieben.

Es wurde gefunden, daß die Verbindungen der allgemeinen Formel I wertvolle pharmakologische Eigenschaften bei guter Verträglichkeit besitzen.

Die erfindungsgemäßen Wirkstoffe können daher zur Prophylaxe und Chemotherapie von lokalen und systemischen Infektionen in der Human- und Tiermedizin verwendet werden. Als Krankheiten, die durch die erfindungsgemäßen Verbindungen verhindert bzw. geheilt werden können, seien beispielsweise solche der Atmungswege, des Rachenraumes und der Harnwege genannt; die Verbindungen wirken insbesondere gegen Pharyngitis, Pneumonie, Peritonitis, Pyelonephritis, Otitis, Cystitis, Endocarditis, Bronchitis, Arthritis und allgemeine systemische Infektionen. Weiter können diese Verbindungen als Stoffe zur Konservierung von anorganischen oder organischen Materialien verwendet werden, besonders von organischen Materialien, wie Polymeren, Schmiermittel, Farben, Fasern, Leder, Papier und Holz sowie von Lebensmitteln.

Dieses wird dadurch ermöglicht, daß die Verbindungen der allgemeinen Formel I sowohl in vitro als auch in vivo gegen schädliche Mikroorganismen, insbesondere gegen grampositive und gramnegative Bakterien und bakterienähnliche Mikroorganismen sehr stark wirken, wobei sie sich besonders durch ein breites Wirkungsspektrum auszeichnen.

Mit diesen Cephalosporinderivaten können beispielsweise lokale und/oder systemische Erkrankungen behandelt und/oder verhindert werden, die durch die folgenden Erreger oder durch Mischungen der folgenden Erreger verursacht werden:

Micrococcaceae, wie Staphylokokken;
Lactobacteriaceae, wie Streptokokken;
Neisseriaceae, wie Neisserien;
Corynebacteriaceae, wie Corynebakterien;
Enterobacteriaceae, wie Escherichia-Bakterien der Coli-Gruppe;
Klebsiella-Bakterien, z. B. K. pneumoniae;
Proteae-Bakterien der Proteus-Gruppe, z. B. Proteus vulgaris;
Salmonella-Bakterien, z. B. S. thyphimurium;
Shigella-Bakterien, z. B. Shigella dysenteriae;
Pseudomonas-Bakterien, z. B. Pseudomonas aeruginosa;
Aeromonas-Bakterien, z. B. Aeromonas lique faciens;
Spirillaceae, wie Vibrio-Bakterien, z. B. Vibrio cholerae;
Parvobacteriaceae oder Brucellaceae, wie Pasteurella-Bakterien;
Brucella-Bakterien, z. B. Brucella abortus;
Haemophilus-Bakterien, z. B. Haemophilus influenzae;
Bordetella-Bakterien, z. B. Bordetella pertussis;
Moraxella-Bakterien, z. B. Moraxella lacunata;
Bacteroidaceae, wie Bacteroides-Bakterien;
Fusiforme-Bakterien, z. B. Fusobacterium fusiforme;
Spaerophorus-Bakterien, z. B. Spaerophorus necrophorus;

Bacillaceae, wie aerobe Sporenbildner, z. B. Bacillus anthracis;
anaerobe Sporenbildner-Chlostridien, z. B. Chlostridium perfringens;
Spirochaetaceae, wie Borrelia-Bakterien;
Treponema-Bakterien, z. B. Treponema pallidum;
Leptospira-Bakterien, wie Leptospira interrogans.

Die obige Aufzählung von Erregern ist lediglich beispielhaft und keineswegs beschränkend aufzufassen.

Im folgenden werden einige typische, besonders gut wirksame Verbindungen gemäß der vorliegenden Erfindung aufgezählt:

Natrium-7β-{D-α[3-(4-hydroxy-2-(3'-pyridylmethylamino)-5-pyrimidinyl)-ureido]-p-hydroxyphenylacetamido}-3-[(1-(2'-hydroxyäthyl)-tetrazol-5-yl)-thiomethyl]-ceph-3-em-4-carboxylat

Natrium-7β-{D-α-[3-(2-(5'-aminosulfonyl-2'-thienylmethylamino)-4-hydroxy-5-pyrimidinyl)-ureido]-p-hydroxyphenylacetamido}-3-[(1-(2'-hydroxyäthyl)-tetrazol-5-yl)-thiomethyl]-ceph-3-em-4-carboxylat

Natrium-7β-{D-α-[3-(2-(5'-aminosulfonyl-2'-thienylmethylamino)-4-hydroxy-5-pyrimidinyl)-ureido]-p-hydroxyphenylacetamido}-3-[(1-vinyl-tetrazol-5-yl)-thiomethyl]-ceph-3-em-4-carboxylat

Natrium-7β-{D,L-α-[3-(2-(5'-aminosulfonyl-2'-thienyl-methyl-amino)-4-hydroxy-5-pyrimidinyl)-ureido]-2-thienylacetylamido}-3-[(2'-hydroxyäthyl)-tetrazol-5-yl)-thiomethyl]-ceph-3-em-4-carboxylat

Natrium-7β-{D-α-[3-(4-hydroxy-2-(4'-methyl-2'-imidazolyl-methylamino)-5-pyrimidinyl)-ureido]-p-hydroxyphenyl-acetamido}-3-([1-(2'-hydroxyäthyl)-tetrazol-5-yl)-thiomethyl]-ceph-3-em-4-carboxylat

Natrium-7β-{D-α-[3-(2-(5'-aminosulfonyl-2'-thienylmethyl-amino)-4-hydroxy-5-pyrimidinyl)-ureido]-p-hydroxyphenyl-acetamido}-3-[(1-(2'-aminocarbonyläthyl)-tetrazol-5-yl)-thio-methyl]-ceph-3-em-4-carboxylat

Natrium-7β{-D-α-[3-(2-(2'-furylmethylamino)-4-hydroxy-5-pyrimidinyl)-ureido]-p-hydroxyphenylacetamido}-3-[(1-(2'-methyl-sulfonyläthyl)-tetrazol-5-yl)-thiomethyl]-ceph-3-em-4-carboxylat

Die Wirksamkeit der erfindungsgemäßen β-Lactam-Antibiotika läßt sich durch folgende Untersuchungen beispielhaft demonstrieren:

## 1. In vitro-Versuche

Für die Untersuchungen wurde die Methode des Reihenverdünnungstests im Mikrotitersystem angewandt. Die Prüfung der Substanzen auf Bakteriostase erfolgte in flüssigem Medium. Es wurden die Bakteriostasewirkung bei folgenden Konzentrationen untersucht:

128, 64, 32, 16, 8, 4, 2, 1, 0,5, 0,25, 0,12, 0,06 µg/ml. Es wurde ein Nährboden folgender Zusammensetzung benutzt: 10 g Pepton, 8 g Fleischextrakt-Oxoid, 3 g Natriumchlorid, 2 g sek. Natriumphosphat werden mit dest. Wasser auf 100 ml aufgefüllt (pH 7,2—7,4). Das Alter der Primärkulturen betrug etwa 20 Stunden.

Die Einstellung der Keimsuspension erfolgte am Photometer (nach Eppendorf) (Reagenzglas-Durchmesser 14 mm, Filter 546 nm) anhand der Trübung einer Bariumsulfat-Vergleichssuspension, die durch eine Bariumsulfat-Aufschwemmung erzeugt wurde, welche durch die Zugabe von 3,0 ml 1 %ige Barium-chloridlösung in 97 ml 1 %iger Schwefelsäure entstand. Nach der Einstellung wurden Streptococcus Aronson im Verhältnis 1 : 15 und die übrigen Testkeime im Verhältnis 1 : 1500 mit einer Kochsalzlösung weiter verdünnt.

16 mg der jeweiligen Substanz wurden in 10 ml-Meßkolben eingewogen und mit dem Lösungsmittel bis zur Marke aufgefüllt. Die weitere Verdünnungsreihe wurde mit destilliertem Wasser oder dem jeweiligen Lösungsmittel hergestellt.

Die Vertiefungen der Mikrotiterplatten wurden mit 0,2 ml Nährmedium, 0,01 ml der entsprechenden Substanzverdünnung und mit einem Tropfen Keimsuspension (0,01 ml) beschickt und 18 bis 20 Stunden bei 37°C bebrütet. Eine Lösungsmittelkontrolle wurde stets mitgeführt.

Die Ablesung wurde makroskopisch vorgenommen, wobei die jeweilige Grenzkonzentration (= niedrigste noch bakteriostatisch wirksame Konzentration) ermittelt wurde.

Als Testorganismen wurden benützt:

Staphyllococcus aureus SG 511, Escherichia coli ATCC 11 775, Pseudomonas aeruginosa Hamburgensis und Pseudomonas aeruginosa BC 19, Serratia marcescens ATCC 13 880, Klebsiella pneumoniae ATCC 10 031 und BC 6, Proteus mirabilis BC 17, Proteus rettgeri, Enterobacter cloacae ATCC 13 047, E. coli R + TEM (β-Lactamase-Träger).

In der folgenden Tabelle 1 sind die ermittelten minimalen Hemmkonzentrationen (MIC) für typische Vertreter der erfindungsgemäßen Verbindungen aufgeführt:

| | |
|---|---|
| Natrium-7β-{D-α[3-(4-hydroxy-2-(3′-pyridylmethylamino)-5-pyrimidinyl)-ureido]-p-hydroxyphenylacetamido}-3-[(1-(2′-hydroxyäthyl)-tetrazol-5-yl)-thiomethyl]-ceph-3-em-4-carboxylat | = Verbindung A |
| Natrium-7β-{D-α-[3-(2-(5′-aminosulfonyl-2′-thienylmethylamino)-4-hydroxy-5-pyrimidinyl)-ureido]-p-hydroxyphenylacetamido}-3-[(1-(2′-hydroxyäthyl)-tetrazol-5-yl)-thiomethyl]-ceph-3-em-4-carboxylat | = Verbindung B |
| Natrium-7β-{D-α-[3-(2-(5′-aminosulfonyl-2′-thienylmethyl-amino)-4-hydroxy-5-pyrimidinyl)-ureido]-p-hydroxyphenyl-acetamido}-3-[(1-(2′-aminocarbonyläthyl)-tetrazol-5-yl)-thiomethyl]-ceph-3-em-4-carboxylat | = Verbindung C |
| Natrium-7β-{D-α-[3-(2-(2′-furylmethylamino)-4-hydroxy-5-pyrimidinyl)-ureido]-p-hydroxyphenylacetamido}-3-[(1-(2′-methylsulfonyläthyl)-tetrazol-5-yl)-thiomethyl]-ceph-3-em-4-carboxylat | = Verbindung D |

Als Vergleichssubstanz diente Cefuroxim.

Tabelle 1

Minimale Hemmkonzentration in μg/ml

| Verbindungen | Staph. aureus SG 511 | E. coli ATCC 11 775 | Pseud. Hbg. | Pseud. BC 19 | Serr. marcesc. ATCC 13 880 | Kl. pneum. ATCC 10 031 | Kl. pneum. BC 6 | Prot. mirab. BC 17 | Prot. rettg. | Ent. cloacae ATCC 13 047 | E. coli R + TEM |
|---|---|---|---|---|---|---|---|---|---|---|---|
| A | 1 | 0,12 | 8 | 4 | 0,25 | 0,25 | 0,5 | 0,12 | 0,5 | 0,5 | 8 |
| B | 1 | 0,06 | 4 | 2 | 0,25 | 0,12 | 0,25 | 0,12 | 0,25 | 0,25 | 4 |
| C | 1 | 0,12 | 4 | 4 | 0,5 | 0,25 | 0,25 | 0,25 | 0,5 | 0,25 | 4 |
| D | 0,5 | 0,25 | 8 | 8 | 0,5 | 0,25 | 0,5 | 0,25 | 1 | 0,5 | 8 |
| Cefuroxim | 1 | 8 | >128 | >128 | 8 | 2 | 4 | 0,5 | 2 | 32 | 4 |

Die akute Toxizität wurde durch perorale und subcutane Applikation der Verbindungen der Tabelle 2 an der Ratte in steigenden Dosen bestimmt.

Die $LD_{50}$ ist die Dosis, nach deren Applikation 50% der Tiere innerhalb von 8 Tagen sterben. Sämtliche Substanzen zeigten bei oraler Gabe eine $LD_{50}$ von über 4 g/kg, bei subcutaner Gabe eine $LD_{50}$ von über 2 g/kg, d.h. sie sind damit für die Praxis untoxisch.

Eine Reihe von erfindungsgemäßen Verbindungen wurde in vivo bei experimentellen Infektionen an Mäusen untersucht. Man verwendete als pathogene Bakterien E. coli ATCC 11 775. Es wurde eine intraperitoneale Infektion mit 0,2 ml einer Bakteriensuspension (mit 5% Mucin) gesetzt. Dies entspricht etwa $1{,}4 \times 10^6$ Keime E. coli/Maus. Weibliche Mäuse vom Stamm NMRI wurden in Gruppen von jeweils 10 Tieren aufgeteilt, zwei Gruppen blieben unbehandelt, die restlichen Gruppen wurden mit verschiedenen Dosen der jeweiligen erfindungsgemäßen Cephalosporine subcutan zur Bestimmung der $ED_{50}$ (Dosis bei der 50% der Tiere überleben) behandelt. Es wurde einmal eine Stunde nach Setzen der Injektion therapiert.

Der Beobachtungszeitraum betrug in beiden Fällen 7 Tage. Die Ergebnisse dieser Teste mit den Vertretern der Tabelle 1 sind in der Tabelle 2 dargestellt.

Tabelle 2

In vivo Aktivität bei Mäusen

E. coli-Infektion (s. c. Applikation):

| Verbindung | $ED_{50}$ (mg/kg) |
|---|---|
| A | 0,9 |
| B | 0,4 |
| C | 0,7 |
| D | 1–2 |
| Cefuroxim | $>100$ |

Es ist eine weitere Aufgabe der vorliegenden Erfindung, pharmazeutische Mittel zu schaffen, die bei der Behandlung infektiöser Krankheiten sowohl beim Menschen als auch beim Tier wertvoll sind.

Als bevorzugte pharmazeutische Zubereitungen seien Tabletten, Dragees, Kapseln, Granulate, Suppositorien, Lösungen, Suspensionen, Emulsionen, Salben, Gele, Cremes, Puder und Sprays genannt. Vorteilhafterweise wird der Wirkstoff in der Human- oder Tiermedizin oder ein Gemisch der verschiedenen Wirkstoffe der allgemeinen Formel I in einer Dosierung zwischen 5 und 500 vorzugsweise 10–200 mg/kg Körpergewicht je 24 Stunden verabreicht, gegebenenfalls in Form mehrerer Einzelgaben. Eine Einzelgabe enthält den oder die erfindungsgemäßen Wirkstoffe, vorzugsweise in Mengen von etwa 1 bis etwa 250, insbesondere 10 bis 60 mg/kg Körpergewicht. Es kann jedoch erforderlich sein, von den genannten Dosierungen abzuweichen, und zwar in Abhängigkeit von der Art und dem Körpergewicht des zu behandelnden Objektes, der Art und der Schwere der Erkrankung, der Art der Zubereitung und der Applikation des Arzneimittels sowie dem Zeitpunkt bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien verhindert, gebessert und/oder geheilt werden und ebenso eine Förderung des Wachstums und eine Verbesserung der Verwertung des Futters erreicht werden.

Die nachfolgenden Beispiele sollen die Erfindung näher beschreiben:

## Beispiel 1

Natrium-7β-{D-α-[3-(2-(5'-aminosulfonyl-2'-thienylmethyl-amino)-4-hydroxy-5-pyrimidinyl)-ureido]-p-hydroxyphenyl-acetamido}-3-[(1-(2'-hydroxyäthyl)-tetrazol-5-yl)-thiomethyl]-ceph-3-em-4-carboxylat

2,72 g (0,005 Mol) D-α-[3-(2-(5'-Aminosulfonyl-2'-thienyl-methylamino-4-hydroxy-5-pyrimidinyl)-ureido]-p-hydroxy-phenylessigsäure werden in 30 ml trockenem Dimethylformamid gelöst. Man fügt eine Lösung von 2,62 g (0,005 Mol) 7-Amino-3-[(1-(2'-hydroxyäthyl)-tetrazol-5-yl)-thiomethyl]-ceph-3-em-4-carbonsäure-diphenylmethylester in 30 ml trockenem Methylenchlorid hinzu. Zu dieser Lösung werden unter Eiskühlung 1,13 g Dicyclohexylcarbodiimid gegeben und es wird 2 Stunden bei 10°C, und 6 Stunden bei Raumtemperatur gerührt. Ein Dünnschichtchromatogramm zeigt, daß die Ausgangsprodukte nahezu vollständig verschwunden sind. Man filtriert und engt dann das Filtrat im Vakuum zur Trockne ein, rührt zweimal mit jeweils 50 ml Methanol und einmal mit 100 ml Methylenchlorid gut aus. Das zurückbleibende Festprodukt wird abgesaugt und mit Äther gut gewaschen. Zur Entfernung geringfügiger Verunreinigungen, die im Dünnschichtchromatogramm (Methylenchlorid : Methanol 4 : 1) am Startfleck sitzen bleiben, wird über eine Kieselgelsäule chromatographiert. Ausbeute an Ester: 3,46 g (65,5 %).

Das so erhaltene Produkt wird in wenig Methylenchlorid suspendiert und unter Eiskühlung 30 Minuten mit 2 ml Anisol und 10 ml Trifluoressigsäure gerührt, wobei Lösung eintritt. Anschließend werden zweimal 50 ml Toluol hinzugefügt und im Vakuum jeweils zur Trockne eingeengt. Man versetzt mit Äther und saugt ab.

Zur Herstellung des Natriumsalzes löst man in wenig Dimethylformamid, gibt die berechnete Menge Natrium-äthylhexanoat in Methanol zu und versetzt mit Äther. Das ausgefällte Produkt wird abgesaugt, sorgfältig mit Äther gewaschen, und im Vakuum getrocknet.

Ausbeute an Natriumsalz (bezogen auf das eingesetzte Cephalosporinderivat): 2,71 g (61 %).

IR-Spektrum: 1760, 1655, 1615, 1550 $cm^{-1}$;

NMR-Spektrum (DMSO + $CD_3OD$) Signale bei ppm:
3,55 (m, 2 H), 3,75 (m, 2 H), 4,2—4,6 (m, 2 + 2 + 2 H), 4,90 d, 1 H), 5,45 (s, 1 H), 5,65 (d, 1 H), 6,75 (d, 2 H), 7,0 (d, 1 H), 7,25 (d, 2 H), 7,40 (d, 1 H), 8,15 (s, 1 H).

Nach der in Beispiel 1 angegebenen Methode wurden die Cephalosporine (Natriumsalze der allgemeinen Formel I) die in der folgenden Tabelle angegeben sind, synthetisiert:

| Beispiel | A | R | D | IR-Spektrum cm$^{-1}$ | NMR-Signale (DMSO + $CD_3OD$) Signale bei ppm: |
|---|---|---|---|---|---|
| 2 | 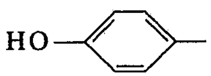 | 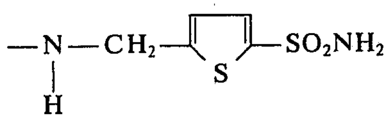 | 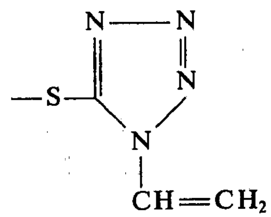 | 1760<br>1655<br>1150 | 3,55 (m, 2 H), 4,30 (m, 4 H), 4,95 (d, 1 H), 5,35–5,90 (m, 4 H), 6,65–7,0 (m, 4 H), 7,25 (d, 2 H), 7,40 (d, 1 H), 8,15 (s, 1 H). |
| 3 | 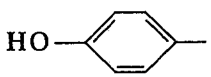 | 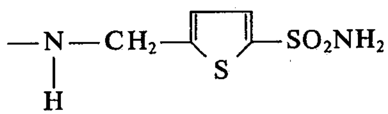 | 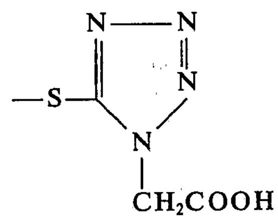 | 1760<br>1640 | 3,0 (m, 2 H), 3,55 (m, 2 H), 4,25 (m, 4 H), 5,0 (d, 1 H), 5,45 (s, 1 H), 5,65 (d, 1 H), 6,75 (d, 2 H), 6,95 (d, 1 H), 7,25 (d, 2 H), 7,40 (d, 1 H), 8,10 (s, 1 H). |
| 4 | 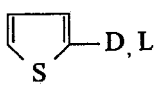 D,L | 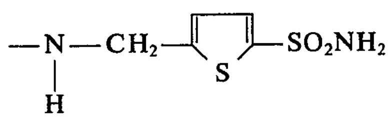 | 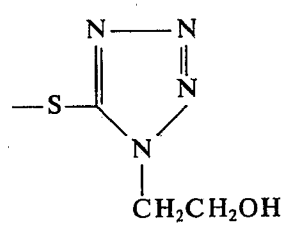 | 1765<br>1660 | 3,55 (m, 2 H), 3,80 (m, 2 H), 4,2–4,6 (m, 6 H), 4,95 (dd, 1 H), 5,55 (dd, 1 H), 5,75 (s, breit, 1 H), 7,0 (m, 4 H), 7,3–7,5 (m, 2 H), 8,15 (s, 1 H). |
| 5 | 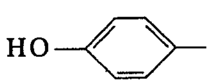 | 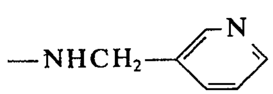 | 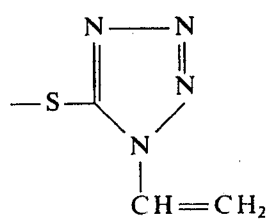 | 1760<br>1655 | 3,60 (m, 2 H), 4,70 (m, 4 H), 4,95 (d, 1 H), 5,30–5,90 (m, 4 H), 6,75 (d, 2 H), 7,30 (m, 3 H), 7,7 (m, 1 H), 8,1 (s, 1 H), 8,5 (m, 2 H). |

## Beispiel 6

Natrium-7-{D-α-[3-(2-(5'-aminosulfonyl)-2'-thienylmethylamino)-4-hydroxy-5-pyrimidinyl)-ureido]-p-hydroxy-phenylacetamido}-3-[(1-(2'-aminoäthyl)-tetrazol-5-yl)-thiomethyl]-ceph-3-em-4-carboxylat

Die Herstellung erfolgte analog Beispiel 1, ausgehend von der dort eingesetzten Ureidocarbonsäure sowie einer äquimolekularen Menge 7-Amino-3-[(1-(2'-t-butoxycarbonylamino-äthyl)-tetrazol-5-yl)-thiomethyl]-ceph-3-em-4-carbonsäure-diphenylmethylester.
Ausbeute nach Abspaltung der Schutzgruppen und Herstellung des Natriumsalzes: 43,5%;
IR-Spektrum: 1760, 1675, 1600 $cm^{-1}$;

NMR-Spektrum (DMSO + $CD_3OD$) Signale bei ppm:
3,15 (m, 2 H), 3,55 (m, 2 H), 4,35 (m, 6 H), 4,95 (d, 1 H), 5,55 (s, 1 H), 5,70 (d, 1 H), 6,75 (d, 2 H), 7,05 (d, 1 H), 7,25 (d, 2 H), 7,4 (d, 1 H), 8,15 (s, 1 H).

## Beispiel 7

Natrium-7-{D-α-[3-(4-hydroxy-2-(4'-methyl-2'-imidazolyl-methylamino)-5-pyrimidinyl)-ureido]-p-hydroxyphenylacetamido}-3-[(1-(2'-acetylaminoäthyl)-tetrazol-5-yl)-thiomethyl]-ceph-3-em-4-carboxylat

Zu einer Lösung von 830 mg (0,002 Mol) der D-α-[3-(4-Hydroxy-2-(4'-methyl-2'-imidazolylmethylamino)-5-pyrimidinyl)-ureido]-p-hydroxyphenylessigsäure, in 20 ml trockenem Dimethylformamid gibt man 0,2 g N-Methylmorpholin. Die Lösung wird auf −15°C abgekühlt und es wird bei dieser Temperatur eine Lösung von 0,22 g Chlorameisensäureäthylester in 5 ml Methylenchlorid zugetropft. Die erhaltene Mischung wird 45 Minuten bei dieser Temperatur gehalten.
Anschließend tropft man bei −15°C eine Lösung von 1,12 g (0,002 Mol) 7-Amino-3-[(1-(2'-acetylaminoäthyl-tetrazol-5-yl)-thiomethyl]-ceph-3-em-4-carbonsäure-diphenylmethylester in 20 ml trockenem Methylenchlorid zu. Man rührt 1 Stunde bei −10°C und läßt dann langsam auf Raumtemperatur kommen. Die Lösung wird im Vakuum zur Trockne eingeengt und, wie in Beispiel 1 beschrieben, weiter behandelt. Die Abspaltung der Esterschutzgruppe erfolgt ebenfalls analog Beispiel 1.
Ausbeute an Natriumsalz: 900 mg (54%);
IR-Spektrum: 1760, 1650, 1610, 1550 $cm^{-1}$;

NMR-Spektrum (DMSO + $CD_3OD$) Signale bei ppm:
1,85 (s, 3 H), 2,1 (s, 3 H), 3,6 (m, 2 + 2 H), 4,2–4,5 (m, 2 + 2 + 2 H), 5,05 (d, 1 H), 5,45 (s, 1 H), 5,65 (d, 1 H), 6,75 (m, 2 + 1 H), 7,25 (d, 2 H), 8,10 (s, 1 H).

## Beispiel 8

7-{D-α-[3-(2-(5'-aminosulfonyl-2'-thienylmethylamino)-4-hydroxy-5-pyrimidinyl)-ureido]-p-hydroxyphenylacetamido}-3-[(1-hydroxysulfonylmethyl-tetrazol-5-yl)-thiomethyl]-ceph-3-em-4-carbonsäure

Aus 495 mg (0,001 Mol) der Ureidocarbonsäure, die in Beispiel 1 eingesetzt wurde, wird analog Beispiel 7 mit N-Methylmorpholin und Chlorameisensäureäthylester das aktivierte Anhydrid hergestellt. Andererseits gibt man zu einer Suspension von 410 mg (0,001 Mol) 7-Amino-3-[(1-hydroxysulfonylmethyltetrazol-5-yl)-thiomethyl]-ceph-3-em-4-carbonsäure in 20 ml wasserfreiem Acetonitril 600 mg N,O-Bistrimethylsilylacetamid, wobei eine Lösung erhalten wird. Diese Lösung wird auf −15°C abgekühlt und zu der oberen Lösung bei dieser Temperatur zugetropft. Danach wird die Mischung bei −10°C 1 Stunde und bei +10°C ebenfalls 1 Stunde lang gerührt. Nach dieser Zeit fügt man 2 ml Methanol zu und filtriert von unlöslichen Material ab. Danach wird das Lösungsmittel im Vakuum entfernt. Der Rückstand wird in 40 ml Wasser aufgenommen und auf pH 7,0 gestellt. Bei diesem pH-Wert wird zweimal mit Essigester ausgeschüttelt. Die wäßrige Phase wird unter Eiskühlung mit verdünnter Salzsäure auf pH 2,9 gestellt, das ausgefallene Produkt wird abgesaugt, mit wenig Wasser gewaschen und im Vakuum getrocknet.
Ausbeute: 445 mg (57%);
IR-Spektrum: 1760, 1660, 1600 $cm^{-1}$;

NMR-Spektrum (DMSO + $CD_3OD$) Signale bei ppm:
3,60 (m, 2 H), 4,25–4,45 (m, 4 H), 5,0 (m, 3 H), 5,55 (s, 1 H), 5,70 (d, 1 H), 6,75 (d, 2 H), 7,0 (d, 1 H), 7,25 (d, 2 H), 7,45 (d, 1 H), 8,15 (s, 1 H).

Analog Beispiel 7 und 8 wurden folgende Cephalosporine der allgemeinen Formel I synthetisiert:

| Beispiel | A | R | D | IR-Spektrum cm⁻¹ | NMR-Signale (DMSO + $CD_3OD$) Signale bei ppm: |
|---|---|---|---|---|---|
| 9 | 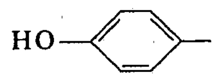 | 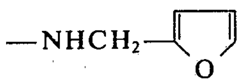 | 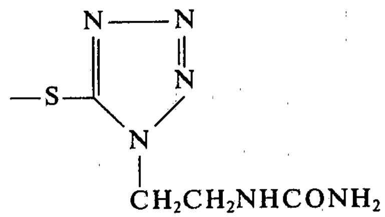 | 1760<br>1660 | 3,0 (m, 2 H), 3,6 (m, 2 H), 4,2–4,5 (m, 6 H), 4,95 (d, 1 H), 5,4 (s, 1 H), 5,65 (d, 1 H), 6,3 (m, 2 H), 6,75 (d, 2 H), 7,25 (d, 2 H), 7,45 (s, 1 H), 8,1 (s, 1 H). |
| 10 | 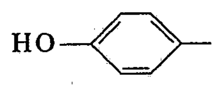 | 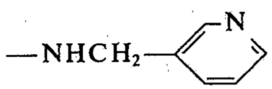 | 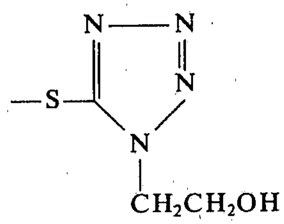 | 1760<br>1650<br>1610 | 3,55 (m, 2 H), 3,8 (m, 2 H), 4,2–4,5 (m, 6 H), 4,95 (d, 1 H), 5,40 (s, 1 H), 5,6 (d, 1 H), 6,75 (d, 2 H), 7,25 (m, 3 H), 7,65 (m, 1 H), 8,1 (s, 1 H), 8,45 (m, 2 H). |
| 11 | 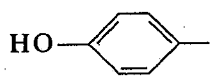 | 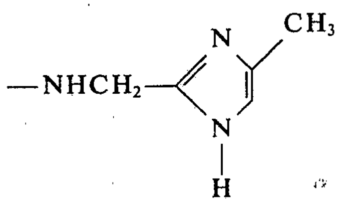 | 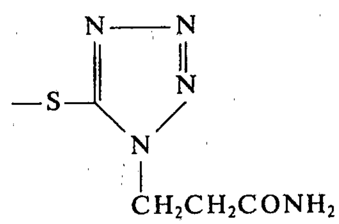 | 1760<br>1665<br>1600 | 2,1 (s, 3 H), 2,5 (m, 2 H), 3,60 (m, 2 H), 4,3 (m, 6 H), 5,0 (d, 1 H), 5,40 (s, 1 H), 5,60 (d, 1 H), 6,75 (m, 3 H), 7,25 (d, 2 H), 8,05 (s, 1 H). |
| 12 | 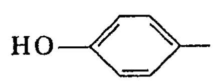 | 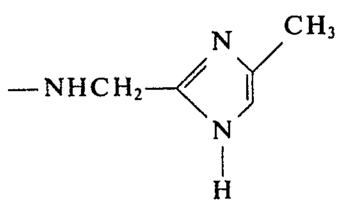 | 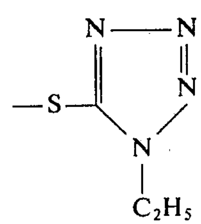 | 1760<br>1650 | 1,0 (t, 3 H), 2,05 (s, 3 H), 3,55 (m, 2 H), 4,3 (m, 6 H), 4,90 (d, 1 H), 5,40 (s, 1 H), 5,65 (d, 1 H), 6,7–6,9 (m, 2 + 1 H), 7,25 (d, 2 H), 8,10 (s, 1 H). |

Fortsetzung

| Beispiel | A | R | D | IR-Spektrum cm$^{-1}$ | NMR-Signale (DMSO + $CD_3OD$) Signale bei ppm: |
|---|---|---|---|---|---|
| 13 | 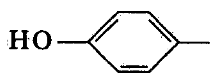 | 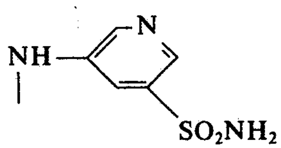 | 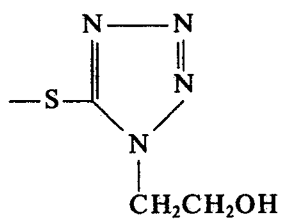 | 1760<br>1660 | 3,5 (m, 2 H), 4,3 (m, 6 H), 4,95 (d, 1 H), 5,45 (s, 1 H), 5,65 (d, 1 H), 6,75 (d, 2 H), 7,25 (d, 2 H), 7,4 (m, 1 H), 8,05 (s, 1 H), 8,2 (m, 2 H). |
| 14 | 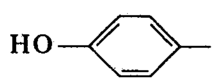 | 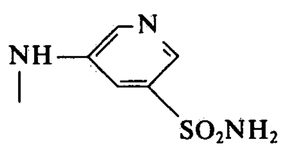 | 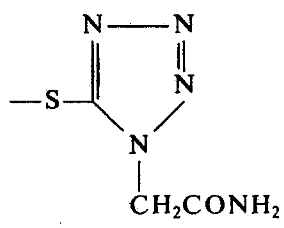 | 1760<br>1655 | 3,65 (m, 2 H), 4,3–4,8 (m, 4 H), 4,95 (d, 1 H), 5,45 (s, 1 H), 5,65 (d, 1 H), 6,75 (d, 2 H), 7,25 (d, 2 H), 7,4 (m, 1 H), 8,05 (s, 1 H), 8,2 (m, 2 H). |

Beispiel 15

Natrium-7-{D-$\alpha$-[3-(2-(5-aminosulfonyl-2'-thienylmethylamino)-4-hydroxy-5-pyrimidinyl)-ureido]-p-hydroxy-phenylacetamido}-3-[(1-(2'-dimethylaminoäthyl)-tetrazol-5-yl)-thiomethyl]-ceph-3-em-carboxylat

Ausgehend von 2,72 g D-$\alpha$-[3-(2-(5'-aminosulfonyl-2'-thienylmethylamino)-4-hydroxy-5-pyrimidinyl)-ureido]-p-hydroxyphenylessigsäure (0,005 Mol) und 1,36 g 7-Aminocephalosporansäure werden analog der Herstellvorschrift des Beispiels 8 2,08 g (57 %) 7-{D-$\alpha$-[3-(5'-Aminosulfonyl-2'-thienylmethylamino)-4-hydroxy-5-pyrimidinyl)-ureido]-p-hydroxy-phenylacetamido}-3-acetoxymethyl-ceph-3-em-4-carbonsäure erhalten.

500 mg dieses Cephalosporins werden in 20 ml einer Phosphorsäurepufferlösung von pH 6,3 zusammen mit 200 mg 1-(2'-Dimethylaminoäthyl)-5-mercapto-tetrazol 6 Stunden unter Stickstoff auf 70°C erwärmt, wobei der pH-Wert zwischen 6 und 6,5 gehalten wird. Nach dieser Zeit wird die Reaktionsflüssigkeit abgekühlt, von etwas Unlöslichem abfiltriert und zweimal mit Essigsäureäthylester ausgeschüttelt. Anschließend wird unter Kühlen Salzsäure bis zu einem pH-Wert von 2,8 zugesetzt. Das ausgefallene Produkt wird abgesaugt, mit wenig Wasser gewaschen und getrocknet. Der Rückstand wird auf die übliche Weise in das Natriumsalz überführt.

Ausbeute: 64 %;
IR-Spektrum: 1760, 1660, 1600 $cm^{-1}$;

NMR-Spektrum (DMSO + $CD_3OD$) Signale bei ppm:
2,35 (s, 6 H), 2,80 (m, 2 H), 3,60 (m, 2 H), 4,30 (m, 6 H), 4,95 (d, 1 H), 5,45 (s, 1 H), 5,65 (d, 1 H), 6,75 (d, 2 H), 7,05 (d, 1 H), 7,30 (d, 2 H), 7,45 (d, 1 H), 8,15 (s, 1 H).

Beispiel 16

Natrium-7$\beta$-{D-$\alpha$-[3-(2-(5'-aminosulfonyl-2'-thienylmethylamino)-4-hydroxy-5-pyrimidinyl)-ureido]-p-hydroxy-phenylacetamido}-3-[(1-methyl-5,6-dioxo-1,3,4-triazin-2-yl)-thiomethyl]-ceph-3-em-4-carboxylat

Die Synthese erfolgte analog Beispiel 15, ausgehend von dem Cephalosporinderivat, das dort eingesetzt wurde sowie 4-Methyl-2-mercapto-5,6-dioxo-1,3,4-triazin.
Ausbeute: 66,5 % (der Theorie);
IR-Spektrum: 1760, 1670, 1600 $cm^{-1}$;

NMR-Spektrum (DMSO + $CD_3OD$) Signale bei ppm:
3,55 (m, 2 H + s, 3 H), 4,35 (m, 4 H), 4,90 (d, 1 H), 5,50 (s, 1 H), 5,65 (d, 1 H), 6,75 (d, 2 H), 7,05 (d, 1 H), 7,25 (d, 2 H), 7,40 (d, 1 H), 8,15 (s, 1 H).

Analog zu Beispiel 15 wurden die Verbindungen der allgemeinen Formel I der folgenden Tabelle hergestellt:

| Beispiel | A | R | D | IR-Spektrum $cm^{-1}$ | NMR-Signale (DMSO + $CD_3OD$) Signale bei ppm: |
|---|---|---|---|---|---|
| 17 | 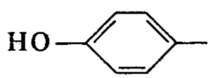 | 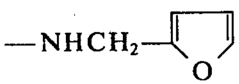 | 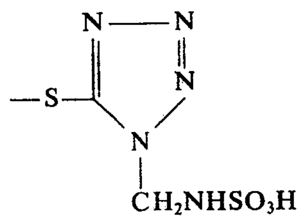 | 1760<br>1655<br>1600 | 3,55 (m, 2 H), 4,35 (m, 4 H),<br>4,8–5,1 (m, 2 + 1 H), 5,4 (s, 1 H),<br>5,6 (d, 1 H), 6,25 (m, 2 H), 6,75 (d, 2 H),<br>7,25 (d, 2 H), 7,40 (s, breit, 1 H),<br>8,1 (s, 1 H). |
| 18 | 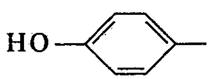 | 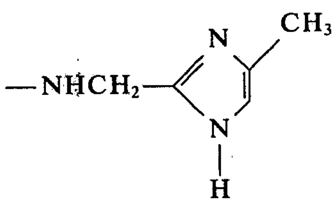 | 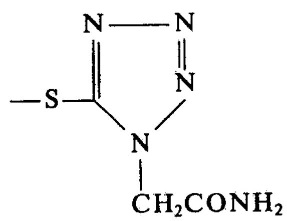 | 1760<br>1650<br>1610 | 2,15 (s, 3 H), 3,65 (m, 2 H),<br>4,35–4,80 (m, 6 H), 4,95 (d, 1 H),<br>5,35 (s, 1 H), 5,60 (d, 1 H), 6,75 (m, 3 H),<br>7,25 (d, 2 H), 8,10 (s, 1 H). |
| 19 | 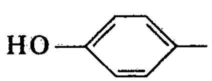 | 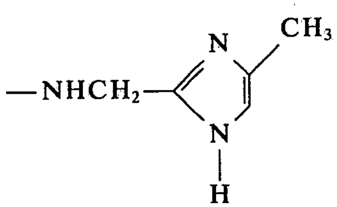 | 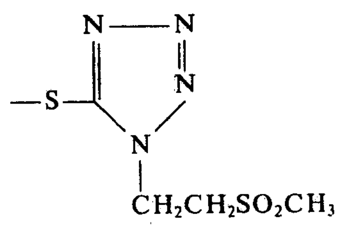 | 1760<br>1660<br>1155 | 2,15 (s, 3 H), 2,85 (s, 3 H),<br>3,5–3,8 (m, 4 H), 4,3 (m, 6 H),<br>4,95 (d, 1 H), 5,40 (s, 1 H), 5,60 (d, 1 H),<br>6,75 (m, 3 H), 7,25 (d, 2 H), 8,10 (s, 1 H). |
| 20 | 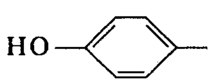 | 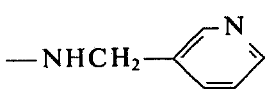 | 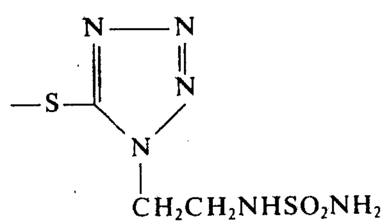 | 1760<br>1650<br>1150 | 3,1–3,7 (m, 4 H), 4,4 (m, 6 H),<br>5,0 (d, 1 H), 5,45 (s, 1 H), 5,65 (d, 1 H),<br>6,75 (d, 2 H), 7,25 (m, 3 H),<br>7,65 (m, 1 H), 8,1 (s, 1 H), 8,50 (m, 2 H). |

Die Verbindungen der allgemeinen Formel I und I' lassen sich in die üblichen pharmazeutischen Anwendungsformen, wie Tabletten, Dragees, Kapseln oder Ampullen einarbeiten. Die Einzeldosis beträgt bei Erwachsenen im allgemeinen zwischen 50 und 1000 mg, vorzugsweise 100 bis 500 mg, die Tagesdosis zwischen 100 und 4000 mg, vorzugsweise 250 bis 2000 mg.

## Beispiel I

Tabletten enthaltend Natrium-7β-{D-α-[3-(4-hydroxy-2-(3'-pyridylmethylamino)-5-pyrimidinyl)-ureido]-p-hydroxyphenyl-acetamido-3-[(1-(2'-hydroxyäthyl)-tetrazol-5-yl)-thiomethyl]-ceph-3-em-4-carboxylat

Ein Gemisch bestehend aus 2 kg Wirksubstanz, 5 kg Lactose, 1,8 kg Kartoffelstärke, 0,1 kg Magnesiumstearat und 0,1 kg Talk wird in üblicher Weise zu Tabletten gepreßt, derart, daß jede Tablette 200 mg Wirkstoff enthält.

## Beispiel II

Dragees enthaltend Natrium-7β-{D-α-[3-(4-hydroxy-2-(3'-pyridyl-methylamino)-5-pyrimidinyl)-ureido]-p-hydroxyphenylacetamido}-3-[(1-(2'-hydroxyäthyl)-tetrazol-5-yl)-thiomethyl]-ceph-3-em-4-carboxylat

Analog Beispiel I werden Tabletten gepreßt, die anschließend in üblicher Weise mit einem Überzug, bestehend aus Zucker, Kartoffelstärke, Talk und Tragant überzogen werden.

## Beispiel III

Kapseln enthaltend Natrium-7β-{D-α-[3-(4-hydroxy-2-(3'-pyridyl-methylamino)-5-pyrimidinyl)-ureido]-p-hydroxyphenyl-acetamido}-3-[(1-(2'-hydroxyäthyl)-tetrazol-5-yl)-thiomethyl]-ceph-3-em-4-carboxylat

5 kg Wirksubstanz werden in üblicher Weise in Hartgelatinekapseln gefüllt, derart, daß jede Kapsel 500 mg des Wirkstoffes enthält.

## Beispiel IV

Trockenampullen enthaltend Natrium-7β-{D-α-[3-(4-hydroxy-2-(3'-pyridylmethylamino)-5-pyrimidinyl)-ureido]-p-hydroxy-phenylacetamido}-3-[(1-(2'-hydroxyäthyl)-tetrazol-5-yl)-thiomethyl]-ceph-3-em-4-carboxylat

In einem aseptischen Bereich wurde 251 g Wirkstoff in 200 ml destilliertem Wasser zur Injektion aufgelöst. Die Lösung wurde durch ein Millipore-Filter (Porengröße 0,22 μm, Produkt der Millipore Corporation, Bedford, USA) filtriert. Die Lösung wurde jeweils in einer Menge von 2,0 ml in 1000 Gläschen (Kapazität 10 ml) eingegossen und es wurde lyophilisiert. Die Gläschen wurden sodann mit einem Kautschukstöpsel und einer Aluminiumkappe verschlossen. Somit wurden Gläschen (Nr. A) jeweils mit 250 mg Wirkstoff erhalten.

Eine physiologische Kochsalzlösung zur Injektion wurde in einer Menge von jeweils 2,0 ml in Ampullen abgefüllt und die Ampullen wurden verschlossen. Auf diese Weise wurden Ampullen (Nr. B) erhalten. Die physiologische Kochsalzlösung in den Ampullen (Nr. B) wurde in die Gläschen (Nr. A) gegossen, wodurch eine injizierbare Zubereitung für die intravenöse Verabreichung erhalten wurde.

Destilliertes Wasser zur Injektion wurde in einer Menge von 20 ml in die Gläschen (Nr. A) gegossen und die Lösung wurde in einer 5 %igen Lösung von Glucose für Injektionen (250 ml) aufgelöst. Auf diese Weise wurden Lösungen für die kontinuierliche Infusion hergestellt.

Analog sind Tabletten, Dragees, Kapseln und Ampullen erhältlich, die einen oder mehrere der übrigen Wirkstoffe der Formel I oder die physiologisch unbedenklichen Salze dieser Verbindungen enthalten.

**Patentansprüche für die Vertragsstaaten BE, CH, DE, FR, IT, LI, LU, NL, SE**

1. Cephalosporine der allgemeinen Formel I

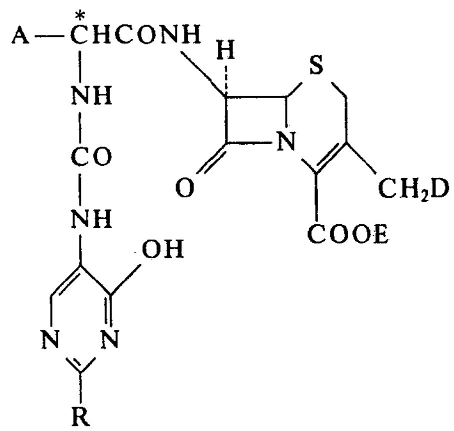

(I)

bzw.

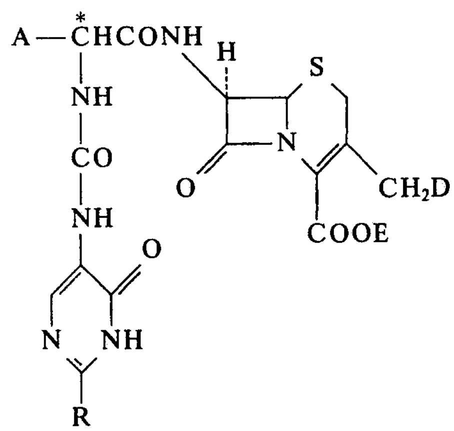

(I')

in welchen A, D, R und E die folgenden Bedeutungen innehaben:

A die Phenyl-, 4-Hydroxyphenyl-, 3,4-Dihydroxyphenyl-, oder die 2- oder 3-Thienylgruppe,

D die Gruppe SHet, wobei Het die 4H-5,6-Dioxo-1,2,4-triazin-3-yl- oder 4-Methyl-5,6-dioxo-1,2,4-triazin-3-ylgruppe, die 1-Vinyl-tetrazol-5-yl- oder 1-Allyltetrazol-5-ylgruppe oder eine Gruppe der allgemeinen Formel II,

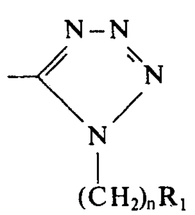

(II)

bedeutet,
in der n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl-, sowie für eine Carbonsäure oder Sulfonsäuregruppe steht, oder in der $(CH_2)_nR_1$ eine Alkylgruppe mit 2 bis 4 Kohlenstoffatomen oder einen 2,3-Dihydroxypropylrest bedeuten,

R eine Gruppe der allgemeinen Formel $NHR_2$, in der $R_2$ eine substituierte oder unsubstituierte 3-Pyridyl-, 5-Pyrimidinyl-, 2-Thienyl-, 2-Furylmethyl-, 2-Thienylmethyl-, 2-Imidazolylmethyl-, 2-Thiazolylmethyl-, 3-Pyridylmethyl- oder eine 5-Pyrimidinylmethylgruppe bedeutet, wobei diese Gruppen durch ein Chloratom, eine Methyl-, Acetylamino-, Hydroxy-, Methylsulfinyl-, Methylsulfonyl-, Aminocarbonyl- oder Aminosulfonylgruppe substituiert sein können,

E ein Wasserstoffatom oder eine in vitro oder in vivo leicht spaltbare Carboxylschutzgruppe und, falls

E ein Wasserstoffatom bedeutet, ihre physiologisch unbedenklichen Salze mit anorganischen oder organischen Basen.

2. Die Verbindungen:

Natrium-7β-{D-α-[3-(4-hydroxy-2-(3'-pyridylmethylamino)-5-pyrimidinyl)-ureido]-p-hydroxyphenylacetamido}-3-[(1-(2'-hydroxyäthyl)-tetrazol-5-yl)-thiomethyl]-ceph-3-em-4-carboxylat
Natrium-7β-{D-α-[3-(2-(5'-aminosulfonyl-2'-thienylmethylamino)-4-hydroxy-5-pyrimidinyl)-ureido]-p-hydroxyphenylacetamido}-3-[(1-(2'-hydroxyäthyl)-tetrazol-5-yl)-thiomethyl]-ceph-3-em-4-carboxylat
Natrium-7β-{D-α-[3-(2-(5'-aminosulfonyl-2'-thienylmethylamino)-4-hydroxy-5-pyrimidinyl)-ureido]-p-hydroxyphenylacetamido}-3-[(1-vinyl-tetrazol-5-yl)-thiomethyl]-ceph-3-em-4-carboxylat
Natrium-7β-{D,L-α-[3-(2-(5'-aminosulfonyl-2'-thienylmethyl-amino)-4-hydroxy-5-pyrimidinyl)-ureido]-2-thienylacetylamido}-3-[(1-2'-hydroxyäthyl)-tetrazol-5-yl)-thiomethyl]-ceph-3-em-4-carboxylat
Natrium-7β-{D-α-[3-(4-hydroxy-2-(4'-methyl-2'-imidazolyl-methylamino)-5-pyrimidinyl)-ureido]-p-hydroxyphenyl-acetamido}-3-[(1-(2'-hydroxyäthyl)-tetrazol-5-yl)-thiomethyl]-ceph-3-em-4-carboxylat
Natrium-7β-{D-α-[3-(2-(5'-aminosulfonyl-2'-thienylmethyl-amino)-4-hydroxy-5-pyrimidinyl)-ureido]-p-hydroxyphenyl-acetamido}-3-[(1-(2'-aminocarbonyläthyl)-tetrazol-5-yl)-thiomethyl]-ceph-3-em-4-carboxylat
Natrium-7β{-D-α-[3-(2-(2'-furylmethylamino)-4-hydroxy-5-pyrimidinyl)-ureido]-p-hydroxyphenylacetamido}-3-[(1-(2'-methyl-sulfonyläthyl)-tetrazol-5-yl)-thiomethyl]-ceph-3-em-4-carboxylat

3. Cephalosporine der allgemeinen Formel I bzw. I' gemäß Anspruch 1, dadurch gekennzeichnet, daß als Carboxylschutzgruppe die Benzyl-, Diphenylmethyl-, Trityl-, t-Butyl-, die 2,2,2-Trichloräthyl-, die Trimethylsilylgruppe oder eine Alkanoyloxyalkylgruppe mit 1 bis 5 Kohlenstoffatomen im Alkanoylrest und 1 bis 3 Kohlenstoffatomen im Alkylenrest dient.

4. Arzneimittel, gekennzeichnet durch einen Gehalt an einem oder mehreren Wirkstoffen der allgemeinen Formel I bzw. I' gemäß den Ansprüchen 1 bis 3 neben den üblichen Träger- und/oder Hilfsstoffen.

5. Verfahren zur Herstellung von Cephalosporinen gemäß Anspruch 1, dadurch gekennzeichnet, daß

a) ein 7-Aminocephalosporansäurederivat der allgemeinen Formel III,

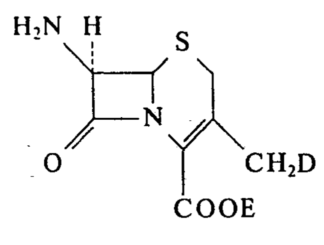

(III)

in der D und E wie in Anspruch 1 definiert sind, mit einer Ureidocarbonsäure der allgemeinen Formel IV,

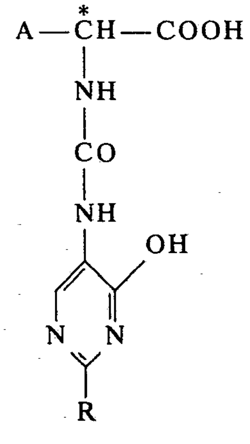

(IV)

in der A und R die in Anspruch 1 genannten Bedeutungen besitzen, oder mit ihren Salzen oder reaktiven Derivaten, zwischen −40° und +40°C in Gegenwart eines Lösungsmittels und, gegebenenfalls, einer Base umgesetzt wird, oder

b) ein Cephalosporansäurederivat der allgemeinen Formel V oder ihre Salze mit anorganischen oder organischen Basen

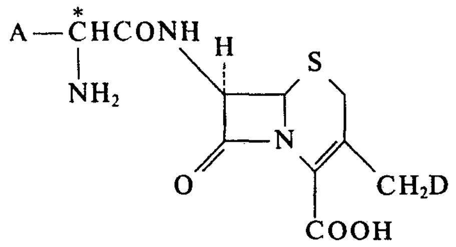

(V)

in der A und D wie in Anspruch 1 definiert sind, mit einem Pyrimidinderivat der allgemeinen Formel VI,

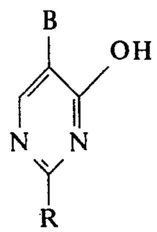

(VI)

in der R wie in Anspruch 1 definiert ist und B die Gruppe −NCO oder ein reaktives Derivat der Gruppe −NHCOOH bzw. die Gruppen −NHCOCl, −NHCOBr oder

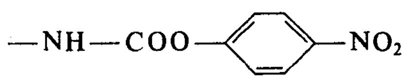

bedeutet, oder mit Gemischen solcher Pyrimidine der allgemeinen Formel VI, in der B teils die eine und teils die andere der vorstehend genannten Bedeutungen besitzt, in einem Lösungsmittel und einem pH-Bereich zwischen 2,0 und 9,0 bei Temperaturen zwischen −20°C und +50°C umgesetzt wird, oder

c) eine Verbindung der allgemeinen Formel VII

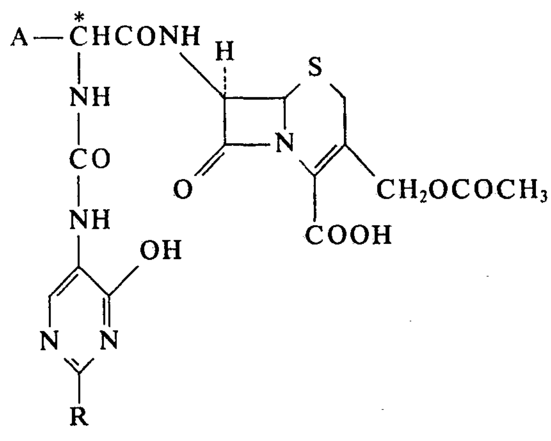

(VII)

in der A und R die in Anspruch 1 angegebenen Bedeutungen besitzen, mit einer Verbindung der allgemeinen Formel VIII

Het—S—M (VIII)

in der Het wie in Anspruch 1 definiert ist und M für ein Wasserstoffatom oder ein Alkalimetall oder ein Erdalkalimetall steht, in einem Lösungsmittel bei Temperaturen zwischen 0° und 100°C in einem pH-Bereich zwischen 2 und 10 zu einer Verbindung der allgemeinen Formel I bzw. I', in der D die Gruppe −SHet und E ein Wasserstoffatom bedeuten, umgesetzt wird, und, falls eine Verbindung der allgemeinen Formel I bzw. I' entsteht, in der E eine in vitro oder in vivo leicht abspaltbare Schutzgruppe darstellt, gegebenenfalls diese Schutzgruppe anschließend abgespalten wird und/ oder eine Verbindung der allgemeinen Formel I bzw. I', in der E ein Wasserstoffatom bedeutet, gegebenenfalls in ihre in vivo oder in vitro leicht spaltbare Ester oder, mittels anorganischer oder organischer Basen, in ihre Salze überführt wird.

6. Verfahren gemäß Anspruch 5 a, dadurch gekennzeichnet, daß man als reaktive Derivate der Ureidocarbonsäuren der allgemeinen Formel IV deren Säureanhydride, deren reaktive Ester, deren reaktive Amide, deren Säurehalogenide oder Säureazide und als 7-Aminocephalosporansäurederivate der allgemeinen Formel III solche Verbindungen verwendet, bei welchen E die eingangs erwähnten Bedeutungen mit Ausnahme der eines Wasserstoffs besitzt, und die Umsetzung in einem Lösungsmittel, gegebenenfalls in Anwesenheit einer Base und/oder eines Kondensationsmittels erfolgt, oder daß man eine Ureidocarbonsäure der allgemeinen Formel IV selbst oder ihre Salze mit den Verbindungen der allgemeinen Formel III in Gegenwart eines Kondensationsmittels umsetzt.

7. Verfahren gemäß Anspruch 5 b, dadurch gekennzeichnet, daß eine Verbindung der allgemeinen Formel V oder eines ihrer Salze mit anorganischen oder organischen Basen mit einer Verbindung der allgemeinen Formel VI,

a) in Wasser oder in mit Wasser mischbaren Lösungsmitteln in Gegenwart von Wasser in einem pH-Bereich von 6,5 bis 8,0 oder
b) in wasserfreien Lösungsmitteln oder
c) in einem Gemenge aus Wasser und mit Wasser nicht mischbaren Lösungsmitteln in einem pH-Bereich zwischen 6,5 und 8,0

umgesetzt wird, oder daß eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI in einem wasser- und hydroxylgruppenfreien bzw. aprotischen Lösungsmittel, gegebenenfalls in Gegenwart einer Base, umgesetzt wird.

8. Verfahren gemäß Anspruch 5 c, dadurch gekennzeichnet, daß die Umsetzung in einem stark polaren Lösungsmittel bei einem pH-Wert von 4 bis 8 durchgeführt wird.

**Patentansprüche für den Vertragsstaat AT**

1. Verfahren zur Herstellung von Cephalosporinen der allgemeinen Formel I

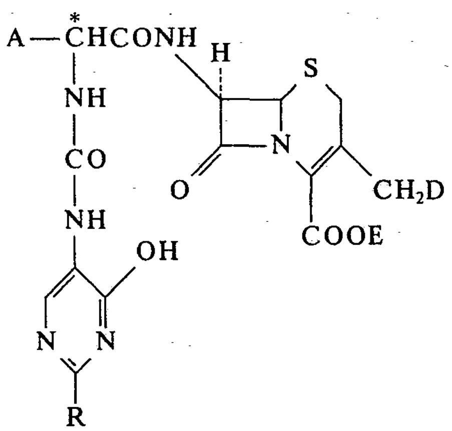

(I)

bzw.

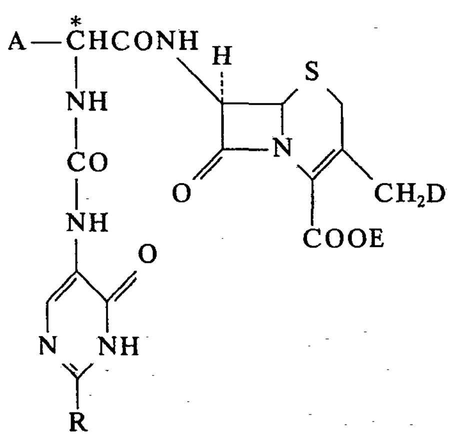

(I')

in welchen A, D, R und E die folgenden Bedeutungen innehaben:

A die Phenyl-, 4-Hydroxyphenyl-, 3,4-Dihydroxyphenyl-, oder die 2- oder 3-Thienylgruppe,

D die Gruppe SHet, wobei Het die 4H-5,6-Dioxo-1,2,4-triazin-3-yl- oder 4-Methyl-5,6-dioxo-1,2,4-triazin-3-ylgruppe, die 1-Vinyl-tetrazol-5-yl- oder 1-Allyltetrazol-5-ylgruppe oder eine Gruppe der allgemeinen Formel II,

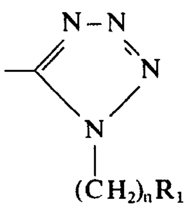

(II)

bedeutet,
in der n die Zahlen 1 bis 3 bedeutet und $R_1$ für die Hydroxygruppe, die Amino-, Dimethylamino-, Acetylamino-, Aminocarbonyl-, Aminocarbonylamino-, Aminosulfonyl-, Aminosulfonylamino-, Methylcarbonyl-, Methylsulfonylamino-, Cyano-, Hydroxysulfonylamino-, Methylsulfonyl-, Methylsulfinyl- sowie für eine Carbonsäure- oder Sulfonsäuregruppe steht, weiter kann $(CH_2)_nR_1$ eine Alkylgruppe mit 2 bis 4 Kohlenstoffatomen oder einen 2,3-Dihydroxypropylrest bedeuten,

R eine Gruppe der allgemeinen Formel $NHR_2$, in der $R_2$ eine substituierte oder unsubstituierte 3-Pyridyl-, 5-Pyrimidinyl-, 2-Thienyl-, 2-Furylmethyl-, 2-Thienylmethyl-, 2-Imidazolylmethyl-, 2-Thiazolylmethyl-, 3-Pyridylmethyl- oder eine 5-Pyrimidinylmethylgruppe bedeutet, wobei diese Gruppen durch ein Chloratom, eine Methyl-, Acetylamino-, Hydroxy-, Methylsulfinyl-, Methylsulfonyl-, Aminocarbonyl- oder Aminosulfonylgruppe substituiert sein können,

E ein Wasserstoffatom oder eine in vitro oder in vivo leicht abspaltbare Carboxylschutzgruppe und, falls E ein Wasserstoffatom bedeutet, von deren physiologisch verträglichen Salzen mit anorganischen oder organischen Basen, dadurch gekennzeichnet, daß

a) ein 7-Aminocephalosporansäurederivat der allgemeinen Formel III,

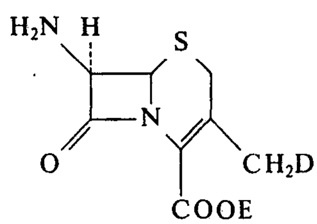

(III)

in der D und E wie oben erwähnt definiert sind, mit einer Ureidocarbonsäure der allgemeinen Formel IV,

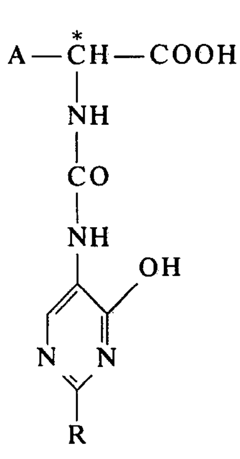

(IV)

in der A und R die oben genannten Bedeutungen besitzen, oder mit ihren Salzen oder reaktiven Derivaten, zwischen −40° und +40°C in Gegenwart eines Lösungsmittels und, gegebenenfalls, einer Base umgesetzt wird, oder

b) ein Cephalosporansäurederivat der allgemeinen Formel V oder ihre Salze mit anorganischen oder organischen Basen

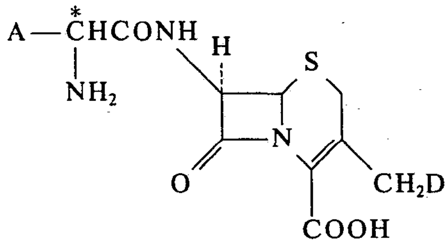

(V)

in der A und D wie oben genannt definiert sind, mit einem Pyrimidinderivat der allgemeinen Formel VI,

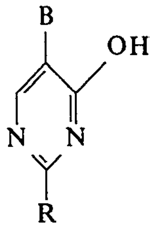

(VI)

in der R wie oben definiert ist und B die Gruppe −NCO oder ein reaktives Derivat der Gruppe −NHCOOH bzw. die Gruppen −NHCOCl, −NHCOBr oder

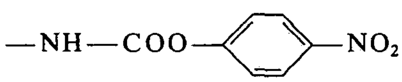

bedeutet, oder mit Gemischen solcher Pyrimidine der allgemeinen Formel VI, in der B teils die eine und teils die andere der vorstehend genannten Bedeutungen besitzt, in einem Lösungsmittel und einem pH-Bereich zwischen 2,0 und 9,0 bei Temperaturen zwischen −20°C und +50°C umgesetzt wird, oder

c) eine Verbindung der allgemeinen Formel VII

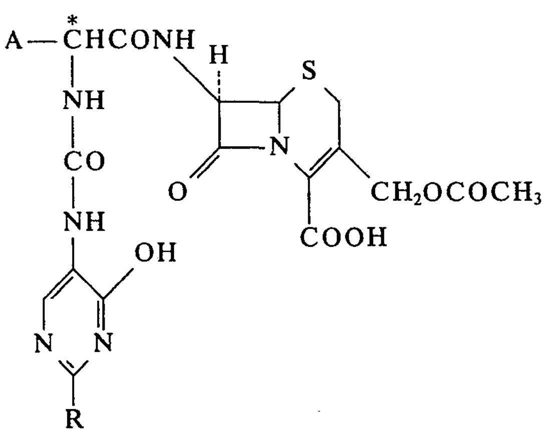

(VII)

in der A und R die oben angegebenen Bedeutungen besitzen, mit einer Verbindung der allgemeinen Formel VIII

Het—S—M (VIII)

in der Het wie eingangs erwähnt definiert ist und M für ein Wasserstoffatom oder ein Alkalimetall oder ein Erdalkalimetall steht, in einem Lösungsmittel bei Temperaturen zwischen 0° und 100°C in einem pH-Bereich zwischen 2 und 10 zu einer Verbindung der allgemeinen Formel I bzw. I', in der D die Gruppe −SHet und E ein Wasserstoffatom bedeuten, umgesetzt wird, und, falls eine Verbindung der allgemeinen Formel I bzw. I' entsteht, in der E eine in vitro oder in vivo leicht abspaltbare Schutzgruppe darstellt, gegebenenfalls diese Schutzgruppe anschließend abgespalten wird und/oder eine Verbindung der allgemeinen Formel I bzw. I', in der E ein Wasserstoffatom bedeutet, gegebenenfalls in ihre in vivo oder in vitro leicht spaltbare Ester oder, mittels anorganischer oder organischer Basen, in ihre Salze überführt wird.

2. Verfahren gemäß Anspruch 1 a, dadurch gekennzeichnet, daß man als reaktive Derivate der Ureidocarbonsäuren der allgemeinen Formel IV deren Säureanhydride, deren reaktive Ester, deren reaktive Amide, deren Säurehalogenide oder Säureazide und als 7-Aminocephalosporansäurederivate der allgemeinen Formel III solche Verbindungen verwendet, bei welchen E die eingangs erwähnten Bedeutungen mit Ausnahme der eines Wasserstoffs besitzt, und die Umsetzung in einem Lösungsmittel, gegebenenfalls in Anwesenheit einer Base und/oder eines Kondensationsmittels erfolgt, oder daß man eine Ureidocarbonsäure der allgemeinen Formel IV selbst oder ihre Salze mit den Verbindungen der allgemeinen Formel III in Gegenwart eines Kondensationsmittels umsetzt.

3. Verfahren gemäß Anspruch 1 b, dadurch gekennzeichnet, daß eine Verbindung der allgemeinen Formel V oder eines ihrer Salze mit anorganischen oder organischen Basen mit einer Verbindung der allgemeinen Formel VI,

a) in Wasser oder in mit Wasser mischbaren Lösungsmitteln in Gegenwart von Wasser in einem pH-Bereich von 6,5 bis 8,0 oder
b) in wasserfreien Lösungsmitteln oder
c) in einem Gemenge aus Wasser und mit Wasser nicht mischbaren Lösungsmitteln in einem pH-Bereich zwischen 6,5 und 8,0

umgesetzt wird, oder daß eine Verbindung der allgemeinen Formel V, in der das Wasserstoffatom der Carboxylgruppe durch eine Silylgruppe oder eine andere leicht abspaltbare Schutzgruppe ersetzt ist, mit einer Verbindung der allgemeinen Formel VI in einem wasser- und hydroxylgruppenfreien bzw. aprotischen Lösungsmittel, gegebenenfalls in Gegenwart einer Base, umgesetzt wird.

4. Verfahren gemäß Anspruch 1 c, dadurch gekennzeichnet, daß die Umsetzung in einem stark polaren Lösungsmittel bei einem pH-Wert von 4 bis 8 durchgeführt wird.

**Claims for the contracting states BE, CH, DE, FR, IT, LI, LU, NL, SE**

1. Cephalosporins of general formula I

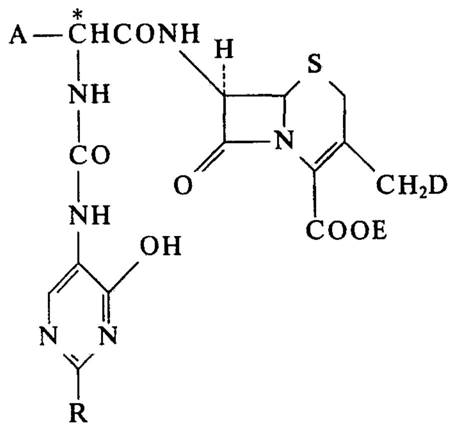

(I)

or

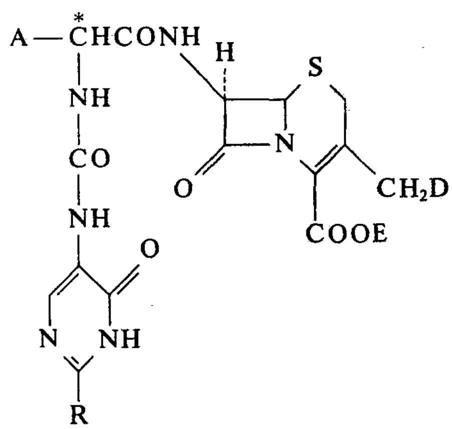

(I')

wherein A, D, R and E have the following definitions:

A represents a phenyl, 4-hydroxyphenyl, 3,4-dihydroxyphenyl or 2- or 3-thienyl group;
D represents the group SHet, wherein Het represents a 4H-5,6-dioxo-1,2,4-triazin-3-yl or 4-methyl-5,6-dioxo-1,2,4-triazin-3-yl group, a 1-vinyl-tetrazol-5-yl or 1-allyltetrazol-5-yl group or a group of formula II

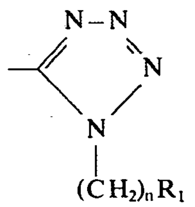

(II)

in which n is a number from 1 to 3 and $R_1$ represents a hydroxy group or an amino, dimethylamino, acetylamino, aminocarbonyl, aminocarbonylamino, aminosulfonyl, aminosulfonylamino, methylcarbonyl, methylsulfonylamino, cyano, hydroxysulfonylamino, methylsulfonyl, methylsulfinyl, carboxylic acid or sulfonic acid group or $(CH_2)_nR_1$ represents an alkyl group with 2 to 4 carbon atoms or a 2,3-dihydroxypropyl group,
R represents a group of the formula $-NHR_2$, wherein $R_2$ represents an optionally substituted 3-pyridyl, 5-pyrimidihyl, 2-thienyl, 2-furylmethyl, 2-thienylmethyl, 2-imidazolylmethyl, 2-thiazolylmethyl, 3-pyridylmethyl or 5-pyrimidinylmethyl group, wherein these groups may be substituted by a chlorine atom or a methyl, acetylamino, hydroxy, methylsulfinyl, methylsulfonyl, aminocarbonyl or aminosulfonyl group;
E represents a hydrogen atom or a carboxyl protecting group easily split off in vitro or in vivo and, if E represents a hydrogen atom, the physiologically acceptable salts thereof with inorganic or organic bases.

2. The compounds:

Sodium 7β-{D-α-[3-(4-hydroxy-2-(3'-pyridylmethylamino)-5-pyrimidinyl)-ureido]-p-hydroxyphenylacetamido}-3-[(1-(2'-hydroxyethyl)-tetrazol-5-yl)-thiomethyl]-ceph-3-em-4-carboxylate

Sodium 7β-{D-α-[3-(2-(5'-aminosulfonyl-2'-thienylmethylamino)-4-hydroxy-5-pyrimidinyl)-ureido]-p-hydroxyphenylacetamido}-3-[(1-(2'-hydroxyethyl)-tetrazol-5-yl)-thiomethyl]-ceph-3-em-4-carboxylate

Sodium 7β-{D-α-[3-(2-(5'-aminosulfonyl-2'-thienylmethylamino)-4-hydroxy-5-pyrimidinyl)-ureido]-p-hydroxyphenylacetamido}-3-[(1-vinyl-tetrazol-5-yl)-thiomethyl]-ceph-3-em-4-carboxylate

Sodium 7β-{D,L-α-[3-(2-(5'-aminosulfonyl-2'-thienylmethylamino)-4-hydroxy-5-pyrimidinyl)-ureido]-2-thienylacetamido}-3-[(1-(2'-hydroxyethyl)-tetrazol-5-yl)-thiomethyl]-ceph-3-em-4-carboxylate

Sodium 7β-{D-α-[3-(4-hydroxy-2-(4'-methyl-2'-imidazolylmethylamino)-5-pyrimidinyl)-ureido]-p-hydroxyphenylacetamido}-3-[(1-(2'-hydroxyethyl)-tetrazol-5-yl)-thiomethyl]-ceph-3-em-4-carboxylate

Sodium 7β-{D-α-[3-(2-(5'-aminosulfonyl-2'-thienylmethylamino)-4-hydroxy-5-pyrimidinyl)-ureido]-p-hydroxyphenylacetamido}-3-[(1-(2'-aminocarbonylethyl)-tetrazol-5-yl)-thiomethyl]-ceph-3-em-4-carboxylate

Sodium 7β-{D-α-[3-(2-(2'-furylmethylamino)-4-hydroxy-5-pyrimidinyl)-ureido]-p-hydroxyphenylacetamido}-3-[(1-(2'-methyl-sulfonylethyl)-tetrazol-5-yl)-thiomethyl]-ceph-3-em-4-carboxylate

3. Cephalosporins of general formula I or I', according to claim 1, characterised in that the benzyl, diphenylmethyl, trityl, t-butyl, 2,2,2-trichloroethyl, trimethylsilyl group or an alkanoyloxyalkyl group with 1 to 5 carbon atoms in the alkanoyl group and 1 to 3 carbon atoms in the alkylene group is used as the protecting group.

4. Pharmaceutical compositions, characterised in that they contain one or more active substances of general formula I or I' as claimed in claims 1 to 3, together with the usual carriers and/or excipients.

5. Process for the preparation of cephalosporins according to claim 1, characterised in that

a) a 7-aminocephalosporanic acid derivative of general formula III,

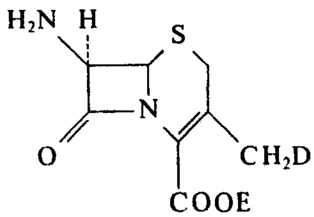

(III)

wherein D and E are as defined in claim 1, is reacted with a ureidocarboxylic acid of general formula IV,

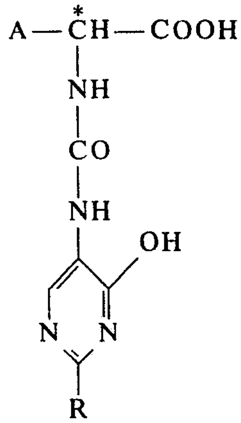

(IV)

wherein A and R are as defined in claim 1, or with the salts or reactive derivatives thereof, at between −40° and +40°C in the presence of a solvent and, optionally, a base, or

b) a cephalosporanic acid derivative of general formula V or the salts thereof with inorganic or organic bases

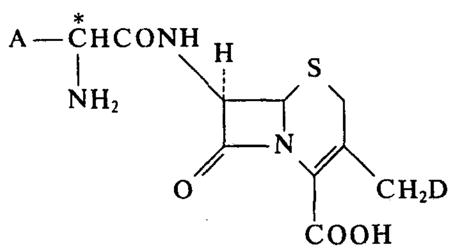

(V)

wherein A and D are as defined in claim 1, is reacted with a pyrimidine derivative of general formula VI

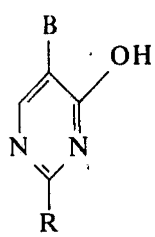

(VI)

wherein R is as defined in claim 1 and B represents the group −NCO or a reactive derivative of the group −NHCOOH or the groups −NHCOCl, −NHCOBr or

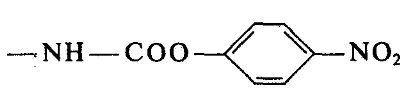

or with mixtures of pyrimidines of general formula VI wherein B is defined partly according to the first and partly according to the second of these two definitions, in a solvent and in a pH range of between 2.0 and 9.0, at temperatures of between −20°C and +50°C, or

c) a compound of general formula VII

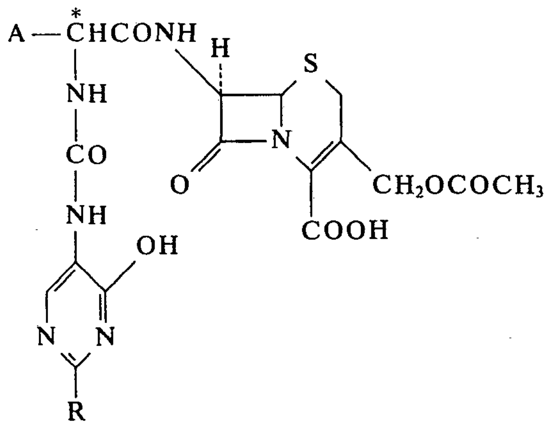

(VII)

wherein A and R are as defined in claim 1, is reacted with a compound of general formula VIII

Het—S—M (VIII)

wherein Het is as defined in claim 1 and M represents a hydrogen atom or an alkali metal or an alkaline earth metal, in a solvent at temperatures between 0° and 100°C in a pH range of between 2 and 10, to yield a compound of general formula I or I' wherein D represents the group —SHet and E represents a hydrogen atom, and, if a compound of general formula I or I' is obtained wherein E represents a protecting group easily split off in vitro or in vivo, this protecting group is subsequently split off, if desired, and/or a compound of general formula I or I' wherein E represents a hydrogen atom is optionally converted into its esters which are easily split off in vivo or in vitro, or, using inorganic or organic bases, converted into the salts thereof.

6. Process as claimed in claim 5 a, characterised in that the reactive derivatives of the ureidocarboxylic acids of general formula IV used are the acid anhydrides, reactive esters, reactive amides, acid halides or acid azides thereof and the compounds used as 7-amino-cephalosporanic acid derivatives of general formula III are compounds wherein E has the meanings given hereinbefore, with the exception of hydrogen, and the reaction is carried out in a solvent, optionally in the presence of a base and/or a condensing agent, or a ureidocarboxylic acid of general formula IV itself or the salts thereof is or are reacted with the compounds of general formula III in the presence of a condensing agent.

7. Process as claimed in claim 5 b, characterised in that a compound of general formula V or one of its salts with inorganic or organic bases is reacted with a compound of general formula VI,

a) in water or in water-miscible solvents in the presence of water in a pH range of 6.5 to 8.0 or
b) in anhydrous solvents or
c) in a mixture of water and water-immiscible solvents in a pH range of 6.5 to 8.0,

or a compound of general formula V wherein the hydrogen atom of the carboxyl group is replaced by a silyl group or another protecting group which is easily split off, is reacted with a compound of general formula VI in an aprotic solvent or a solvent free from water and hydroxyl groups, optionally in the presence of a base.

8. Process as claimed in claim 5 c, characterised in that the reaction is carried out in a strongly polar solvent at a pH of 4 to 8.

**Claims for the contracting state AT**

1. Process for the preparation of cephalosporins of general formula I

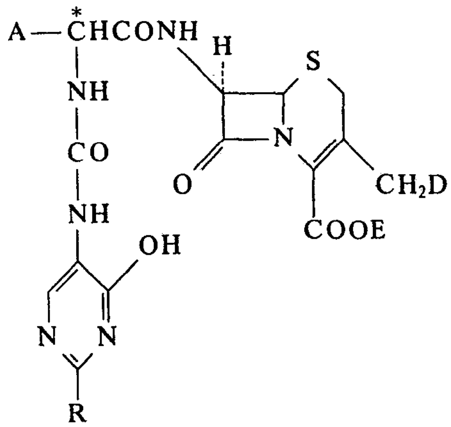

(I)

or

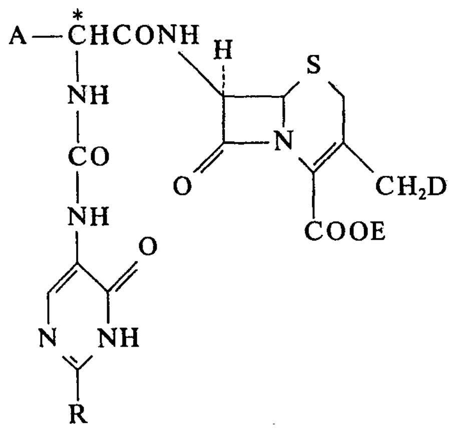

(I')

wherein A, D, R and E have the following definitions:

A represents a phenyl, 4-hydroxyphenyl, 3,4-dihydroxyphenyl or 2- or 3-thienyl group;

D represents the group SHet, wherein Het represents a 4H-5,6-dioxo-1,2,4-triazin-3-yl or 4-methyl-5,6-dioxo-1,2,4-triazin-3-yl group, a 1-vinyl-tetrazol-5-yl or 1-allyltetrazol-5-yl group or a group of formula II

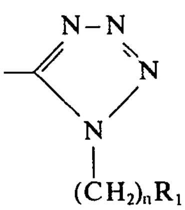

(II)

in which n is a number from 1 to 3 and $R_1$ represents a hydroxy group or an amino, dimethylamino, acetylamino, aminocarbonyl, aminocarbonylamino, aminosulfonyl, aminosulfonylamino, methylcarbonyl, methylsulfonylamino, cyano, hydroxysulfonylamino, methylsulfonyl, methylsulfinyl, carboxylic acid or sulfonic acid group or $(CH_2)_nR_1$ represents an alkyl group with 2 to 4 carbon atoms or a 2,3-dihydroxypropyl group,

R represents a group of the formula $-NHR_2$, wherein $R_2$ represents an optionally substituted 3-pyridyl, 5-pyrimidihyl, 2-thienyl, 2-furylmethyl, 2-thienylmethyl, 2-imidazolylmethyl, 2-thiazolylmethyl, 3-pyridylmethyl or 5-pyrimidinylmethyl group, wherein these groups may be substituted by a chlorine atom or a methyl, acetylamino, hydroxy, methylsulfinyl, methylsulfonyl, aminocarbonyl or aminosulfonyl group;

E represents a hydrogen atom or a carboxyl protecting group easily split off in vitro or in vivo and, if E represents a hydrogen atom, the physiologically acceptable salts thereof with inorganic or organic

bases, characterised in that

a) a 7-aminocephalosporanic acid derivative of general formula III,

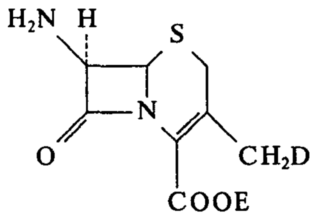

(III)

wherein D and E are as hereinbefore defined, is reacted with a ureidocarboxylic acid of general formula IV,

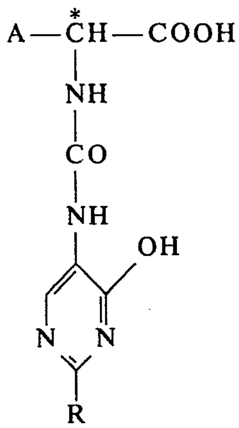

(IV)

wherein A and R are as hereinbefore defined, or with the salts or reactive derivatives thereof, at between −40° and +40°C in the presence of a solvent and, optionally, a base, or

b) a cephalosporanic acid derivative of general formula V or the salts thereof with inorganic or organic bases

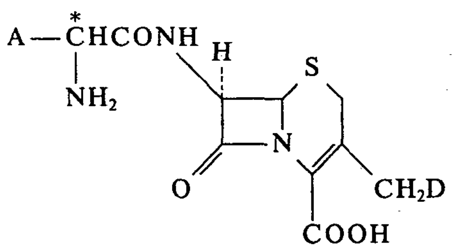

(V)

wherein A and D are as hereinbefore defined is reacted with a pyrimidine derivative of general formula VI

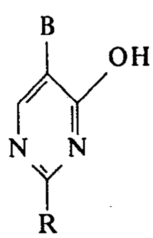

(VI)

wherein R is as hereinbefore defined and B represents the group −NCO or a reactive derivative of the group −NHCOOH or the groups −NHCOCl, −NHCOBr or

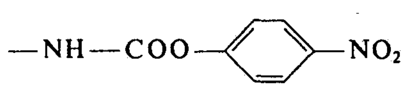

or with mixtures of pyrimidines of general formula VI wherein B is defined partly according to

the first and partly according to the second of these two definitions, in a solvent and in a pH range of between 2.0 and 9.0, at temperatures of between −20°C and +50°C, or

c) a compound of general formula VII

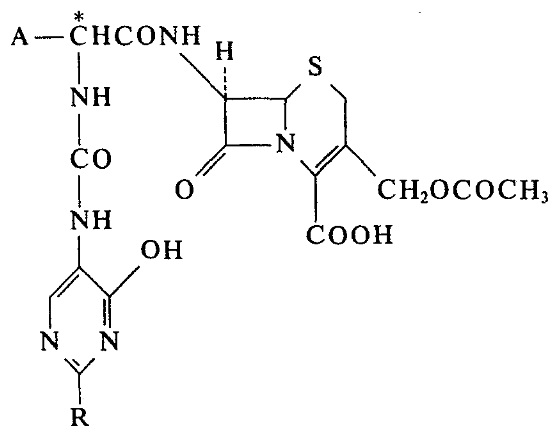

(VII)

wherein A and R are as hereinbefore defined, is reacted with a compound of general formula VIII

Het—S—M (VIII)

wherein Het is as hereinbefore defined and M represents a hydrogen atom or an alkali metal or an alkaline earth metal, in a solvent at temperatures between 0° and 100°C in a pH range of between 2 and 10, to yield a compound of general formula I or I′ wherein D represents the group—SHet and E represents a hydrogen atom, and, if a compound of general formula I or I′ is obtained wherein E represents a protecting group easily split off in vitro or in vivo, this protecting group is subsequently split off, if desired, and/or a compound of general formula I or I′ wherein E represents a hydrogen atom is optionally converted into its esters which are easily split off in vivo or in vitro, or, using inorganic or organic bases, converted into the salts thereof.

2. Process as claimed in claim 1 a, characterised in that the reactive derivatives of the ureidocarboxylic acids of general formula IV used are the acid anhydrides, reactive esters, reactive amides, acid halides or acid azides thereof and the compounds used as 7-amino-cephalosporanic acid derivatives of general formula III are compounds wherein E has the meanings given hereinbefore, with the exception of hydrogen, and the reaction is carried out in a solvent, optionally in the presence of a base and/or a condensing agent, or a ureidocarboxylic acid of general formula IV itself or the salts thereof is or are reacted with the compounds of general formula III in the presence of a condensing agent.

3. Process as claimed in claim 1 b, characterised in that a compound of general formula V or one of its salts with inorganic or organic bases is reacted with a compound of general formula VI,

a) in water or in water-miscible solvents in the presence of water in a pH range of 6.5 to 8.0 or
b) in anhydrous solvents or
c) in a mixture of water and water-immiscible solvents in a pH range of 6.5 to 8.0,

or a compound of general formula V wherein the hydrogen atom of the carboxyl group is replaced by a silyl group or another protecting group which is easily split off, is reacted with a compound of general formula VI in an aprotic solvent or a solvent free from water and hydroxyl groups, optionally in the presence of a base.

4. Process as claimed in claim 1 c, characterised in that the reaction is carried out in a strongly polar solvent at a pH of 4 to 8.

**Revendications pour les Etats contractants BE, CH, DE, FR, IT, LI, LU, NL, SE**

1. Céphalosporines de formule générale I

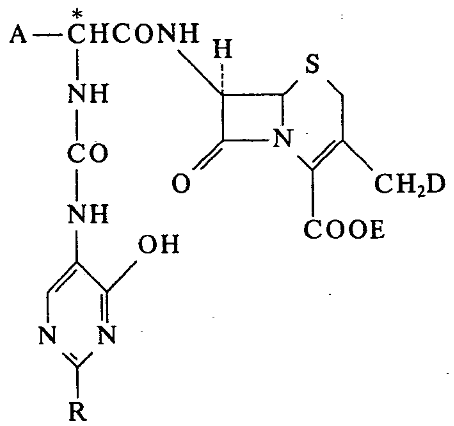

(I)

ou

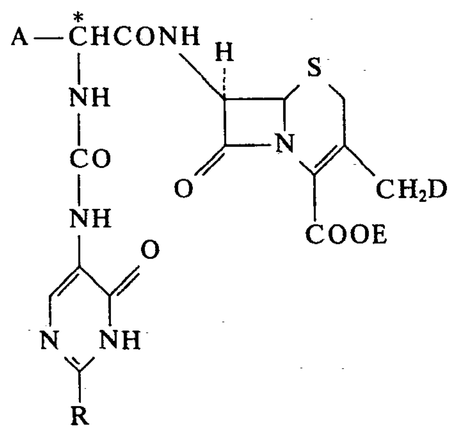

(I')

dans lesquelles A, D, R et E possèdent les significations suivantes:

A représente le groupe phényle, 4-hydroxyphényle, 3,4-dihydroxyphényle ou le groupe 2- ou 3-thiényle,

D représente le groupe SHet, où Het représente le groupe 4H-5,6-dioxo-1,2,4-triazine-3-yle ou 4-méthyl-5,6-dioxo-1,2,4-triazine-3-yle, le groupe 1-vinyl-tétrazol-5-yle ou 1-allyltétrazol-5-yle ou un groupe de formule générale II,

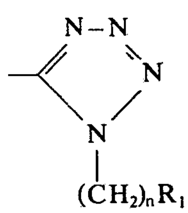

(II)

dans laquelle n représente les nombres 1 à 3 et $R_1$ remplace le groupe hydroxy, le groupe amino, diméthylamino, acétylamino, aminocarbonyle, aminocarbonylamino, aminosulfonyle, aminosulfonylamino, méthylcarbonyle, méthylsulfonylamino, cyano, hydroxysulfonylamino, méthylsulfonyle, méthylsulfinyle, ainsi qu'un groupe acide carboxylique ou acide sulfonique, ou dans laquelle $(CH_2)_nR_1$ représente un groupe alcoyle avec 2 à 4 atomes de carbone ou un radical 2,3-dihydroxypropyle,

R représente un groupe de formule générale $NHR_2$, dans laquelle $R_2$ représente un groupe 3-pyridyle, 5-pyrimidinyle, 2-thiényle, 2-furylméthyle, 2-thiénylméthyle, 2-imidazolylméthyle, 2-thiazolylméthyle, 3-pyridylméthyle ou un groupe 5-pyrimidinylméthyle substitué ou non substitué, ces groupes pouvant être substitués par un atome de chlore, un groupe méthyle, acétylamino, hydroxy, méthylsulfinyle, méthylsulfonyle, aminocarbonyle ou aminosulfonyle,

E représente un atome d'hydrogène ou un groupe protecteur du carboxyle facilement clivable in vitro ou in vivo et, dans le cas où E représente un atome d'hydrogène, leurs sels physiologiquement

inoffensifs avec des bases minérales ou organiques.

2. Les composés:

7β-{D-α-[3-(4-hydroxy-2-(3'-pyridylméthylamino)-5-pyrimidinyl)-uréido]-p-hydroxyphényl-acétamido}-3-[(1-(2'-hydroxyéthyl)-tétrazol-5-yl)-thiométhyl]-ceph-3-ème-4-carboxylate de sosium
7β-{D-α-[3-(2-(5'-aminosulfonyl-2'-thiénylméthylamino)-4-hydroxy-5-pyrimidinyl)-uréido]-p-hydroxyphénylacétamido}-3-[(1-(2'-hydroxyéthyl)-tétrazol-5-yl)-thiométhyl]-ceph-3-ème-4-carboxylate de sodium
7β-{D-α-[3-(2-(5'-aminosulfonyl-2'-thiénylméthylamino)-4-hydroxy-5-pyrimidinyl)-uréido]-p-hydroxyphénylacétamido}-3-[(1-vinyl-tétrazol-5-yl)-thiométhyl]-ceph-3-ème-4-carboxylate de sodium
7β-{D,L-α-[3-(2-(5'-aminosulfonyl-2'-thiényl-méthyl-amino)-4-hydroxy-5-pyrimidinyl)-uréido]-2-thiénylacétylamido}-3-[(1-(2'-hydroxyéthyl)-tétrazol-5-yl)-thiométhyl]-ceph-3-ème-4-carboxylate de sodium
7β-{D-α-[3-(4-hydroxy-2-(4'-méthyl-2'-imidazolyl-méthylamino)-5-pyrimidinyl)-uréido]-p-hydroxyphényl-acétamido}-3-[(1-(2'-hydroxyéthyl)-tétrazol-5-yl)-thiométhyl]-ceph-3-ème-4-carboxylate de sodium
7β-{D-α-[3-(2-(5'-aminosulfonyl-2'-thiénylméthyl-amino)-4-hydroxy-5-pyrimidinyl)-uréido]-p-hydroxyphényl-acétamido}-3-[(1-(2'-aminocarbonyléthyl)-tétrazol-5-yl)-thiométhyl]-ceph-3-ème-4-carboxylate de sodium
7β-{D-α-[3-(2-(2'-furylméthylamino)-4-hydroxy-5-pyrimidinyl)-uréido]-p-hydroxyphényl-acétamido}-3-[(1-(2'-méthyl-sulfonyléthyl)-tétrazol-5-yl)-thiométhyl]-ceph-3-ème-4-carboxylate de sodium.

3. Céphalosporines de formule générale I ou I' selon la revendication 1, caractérisées en ce que le groupe benzyle, diphénylméthyle, trityle, t-butyle, le groupe 2,2,2-trichloroéthyle, le groupe triméthylsilyle ou un groupe alcanoyloxyalcoyle avec 1 à 5 atomes de carbone dans le radical alcanoyle et 1 à 3 atomes de carbone dans le radical alcoylène sert de groupe protecteur du carboxyle.

4. Médicament, caractérisé par une teneur en une ou plusieurs substances actives de formule générale I ou I' selon les revendications 1 à 3 conjointement aux excipients et/ou adjuvants usuels.

5. Procédé pour la préparation de céphalosporines selon la revendication 1, caractérisé en ce que:

a) on fait réagir un dérivé d'acide 7-aminocéphalosporanique de formule générale III,

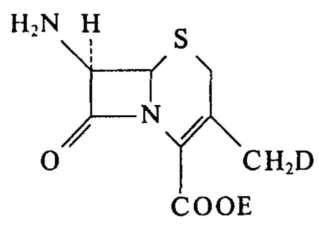

(III)

dans laquelle D et E sont définis comme dans la revendication 1, avec un acide uréidocarboxylique de formule générale IV,

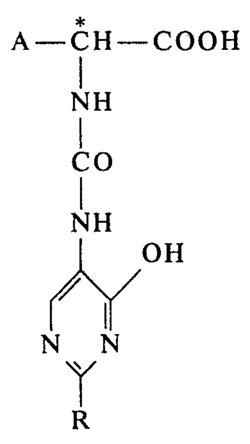

(IV)

dans laquelle A et R possèdent les significations mentionnées dans la revendication 1, ou avec leurs

sels ou dérivés réactifs, entre −40 et +40°C en présence d'un solvant et éventuellement d'une base, ou

b) on fait réagir un dérivé d'acide céphalosporanique de formule générale V ou ses sels avec des bases minérales ou organiques

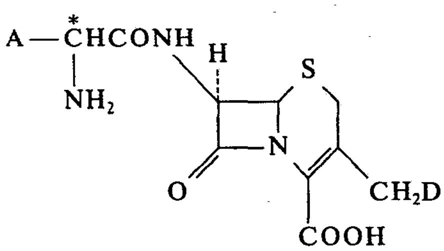

(V)

dans laquelle A et D sont définis comme dans la revendication 1, avec un dérivé de pyrimidine de formule VI,

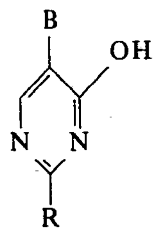

(VI)

dans laquelle R est défini comme dans la revendication 1 et B représente le groupe −NCO ou un dérivé réactif du groupe −NHCOOH ou les groupes −NHCOCl, −NHCOBr ou

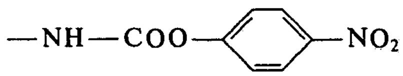

ou avec des mélanges de pyrimidines de formule générale VI, dans laquelle B possède en partie l'une et en partie les autres des significations mentionnées plus haut, dans un solvant et dans un domaine de pH entre 2,0 et 9,0 à des températures entre −20°C et +50°C, ou

c) on fait réagir un composé de formule générale VII

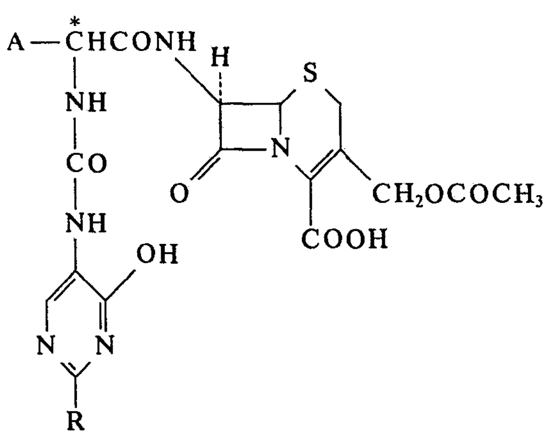

(VII)

dans laquelle A et R possèdent les significations indiquées dans la revendication 1, avec un composé de formule générale VIII

Het—S—M (VIII)

dans laquelle Het est défini comme dans la revendication 1 et M remplace un atome d'hydrogène ou un métal alcalin ou un métal alcalino-terreux, dans un solvant à des températures entre 0° et 100°C dans un domaine de pH entre 2 et 10 pour donner un composé de formule générale I ou respectivement I', dans laquelle D représente le groupe −SHet et E un atome d'hydrogène et, s'il en résulte un composé de formule générale I ou respectivement I' dans laquelle E représente un groupe protecteur facilement clivable in vitro ou in vivo, ce groupe protecteur est éventuellement

clivé ensuite et/ou si on obtient un composé de formule générale I ou I' dans laquelle E représente un atome d'hydrogène, il est éventuellement transformé en ses esters facilement clivables in vivo ou in vitro ou, au moyen de bases minérales ou organiques, en ses sels.

6. Procédé selon la revendication 5 a, caractérisé en ce que l'on utilise en tant dérivés réactifs des acides uréidocarboxyliques de formule générale IV, leurs anhydrides d'acides, leurs esters réactifs, leurs amides réactifs, leurs halogénures d'acides ou leurs azides d'acides et en tant que dérivés d'acides 7-aminocéphalosporaniques de formule générale III des composés dans lesquels E possède les significations indiquées au début à l'exception de celle d'un hydrogène et en ce que la réaction a lieu dans un solvant, éventuellement en présence d'une base et/ou d'un agent de condensation, ou en ce qu'on fait réagir un acide uréidocarboxylique de formule générale IV lui-même ou ses sels avec les composés de formule générale III en présence d'un agent de condensation.

7. Procédé selon la revendication 5 b, caractérisé en ce qu'on fait réagir un composé de formule générale V ou l'un de ses sels avec des bases minérales ou organiques avec un composé de formule générale VI,

a) dans l'eau ou dans des solvants miscibles à l'eau en présence d'eau dans un domaine de pH de 6,5 à 8,0 ou
b) dans des solvants anhydres ou
c) dans un mélange d'eau et de solvants non miscibles à l'eau dans un domaine de pH compris entre 6,5 et 8,0

ou en ce qu'on fait réagir un composé de formule générale V dans laquelle l'atome d'hydrogène du groupe carboxyle est remplacé par un groupe silyle ou un autre groupe protecteur facilement clivable, avec un composé de formule générale VI dans un solvant anhydre et ne contenant pas de groupes hydroxyles ou aprotique, éventuellement en présence d'une base.

8. Procédé selon la revendication 5 c, caractérisé en ce que la réaction est effectuée dans un solvant fortement polaire à une valeur de pH de 4 à 8.

**Revendications pour l'Etat contractant AT**

1. Procédé pour la préparation de céphalosporines de formule générale I

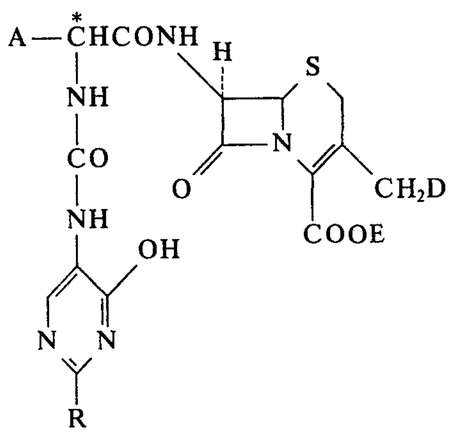

(I)

ou

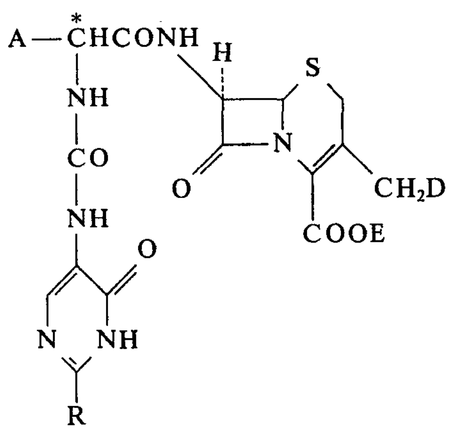

(I')

2. Procédé selon la revendication 1 a, caractérisé en ce que l'on utilise en tant que dérivés réactifs des acides uréidocarboxyliques de formule générale IV, leurs anhydrides d'acides, leurs esters réactifs, leurs amides réactifs, leurs halogénures ou leurs azides d'acides et en tant que dérivés de l'acide 7-amino-céphalosporanique de formule générale III des composés dans lesquels E possède les significations mentionnées au début à l'exception de celle d'un hydrogène, et en ce que la réaction a lieu dans un solvant, éventuellement en présence d'une base et/ou d'un agent de condensation, ou en ce qu'on fait réagir un acide uréidocarboxylique de formule générale IV lui-même ou ses sels avec les composés de formule générale III en présence d'un agent de condensation.

3. Procédé selon la revendication 1 b, caractérisé en ce qu'on fait réagir un composé de formule générale V ou l'un de ses sels avec des bases minérales ou organiques avec un composé de formule générale VI,

a) dans l'eau ou dans des solvants miscibles à l'eau en présence d'eau dans un domaine de pH de 6,5 à 8,0 ou
b) dans des solvants anhydres ou
c) dans un mélange d'eau et de solvants non miscibles à l'eau dans un domaine de pH compris entre 6,5 et 8,0

ou en ce qu'on fait réagir un composé de formule générale V dans laquelle l'atome d'hydrogène du groupe carboxyle est remplacé par un groupe silyle ou un autre groupe protecteur facilement clivable, avec un composé de formule générale VI dans un solvant anhydre et ne contenant pas de groupes hydroxyles ou aprotique, éventuellement en présence d'une base.

4. Procédé selon la revendication 1 c, caractérisé en ce que la réaction est effectuée dans un solvant fortement polaire à une valeur de pH de 4 à 8.

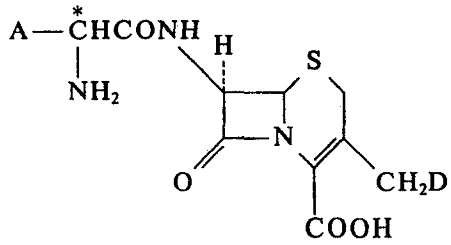

(V)

dans laquelle A et D sont définis comme indiqué plus haut, avec un dérivé de pyrimidine de formule VI,

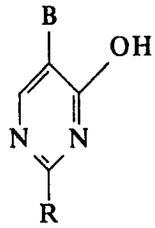

(VI)

dans laquelle R est défini comme indiqué plus haut et B représente le groupe $-NCO$ ou un dérivé réactif du groupe $-NHCOOH$ ou les groupes $-NHCOCl$, $-NHCOBr$ ou

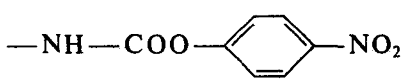

ou avec des mélanges de pyrimidines de formule générale VI, dans laquelle B possède en partie l'une et en partie les autres des significations mentionnées plus haut, dans un solvant et dans un domaine de pH entre 2,0 et 9,0 à des températures entre −20°C et +50°C, ou

c) on fait réagir un composé de formule générale VII

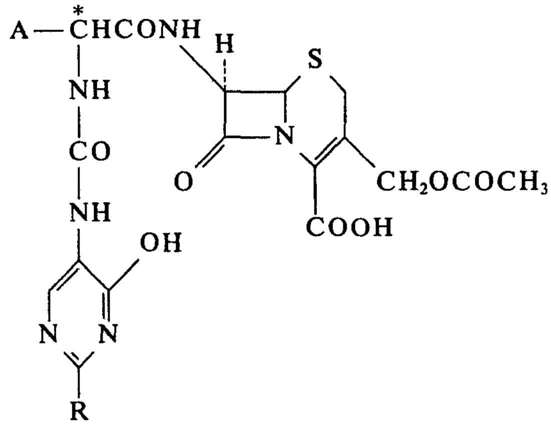

(VII)

dans laquelle A et R possèdent les significations indiquées plus haut, avec un composé de formule générale VIII

Het—S—M (VIII)

dans laquelle Het est défini comme indiqué au début et M remplace un atome d'hydrogène ou un métal alcalin ou un métal alcalino-terreux, dans un solvant à des températures entre 0° et 100°C dans un domaine de pH entre 2 et 10 pour donner un composé de formule générale I ou respectivement I', dans laquelle D représente le groupe $-SHet$ et E un atome d'hydrogène et, s'il en résulte un composé de formule générale I ou respectivement I' dans laquelle E représente un groupe protecteur facilement clivable in vitro ou in vivo, ce groupe protecteur est éventuellement clivé ensuite et/ou si on obtient un composé de formule générale I ou I' dans laquelle E représente un atome d'hydrogène, il est éventuellement transformé en ses esters facilement clivables in vivo ou in vitro ou, au moyen de bases minérales ou organiques, en ses sels.

dans lesquelles A, D, R et E possèdent les significations suivantes:

A représente le groupe phényle, 4-hydroxyphényle, 3,4-dihydroxyphényle ou le groupe 2- ou 3-thiényle,

D représente le groupe SHet, où Het représente le groupe 4H-5,6-dioxo-1,2,4-triazine-3-yle ou 4-méthyl-5,6-dioxo-1,2,4-triazine-3-yle, le groupe 1-vinyl-tétrazol-5-yle ou 1-allyltétrazol-5-yle ou un groupe de formule générale II,

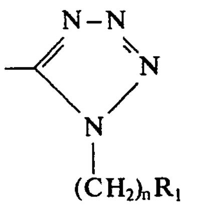

(II)

dans laquelle n représente les nombres 1 à 3 et $R_1$ remplace le groupe hydroxy, le groupe amino, diméthylamino, acétylamino, aminocarbonyle, aminocarbonylamino, aminosulfonyle, aminosulfonyl amino, méthylcarbonyle, méthylsulfonylamino, cyano, hydroxysulfonylamino, méthylsulfonyle, méthylsulfinyle, ainsi qu'un groupe acide carboxylique ou acide sulfonique, de plus $(CH_2)_nR_1$ peut représenter un groupe alcoyle avec 2 à 4 atomes de carbone ou un radical 2,3-dihydroxypropyle.

R représente un groupe de formule générale $NHR_2$, dans laquelle $R_2$ représente un groupe 3-pyridyle, 5-pyrimidinyle, 2-thiényle, 2-furylméthyle, 2-thiénylméthyle, 2-imidazolylméthyle, 2-thiazolylméthyle, 3-pyridylméthyle ou un groupe 5-pyrimidinylméthyle, substitué ou non substitué, ces groupes pouvant être substitués par un atome de chlore, un groupe méthyle, acétylamino, hydroxy, méthylsulfinyle, méthylsulfonyle, aminocarbonyle ou aminosulfonyle,

E représente un atome d'hydrogène ou un groupe protecteur du carboxyle facilement clivable in vitro ou in vivo et, dans le cas où E représente un atome d'hydrogène, de leurs sels physiologiquement supportables avec des bases minérales ou organiques, caractérisé en ce que

a) on fait réagir un dérivé d'acide 7-aminocephalosporanique de formule générale III,

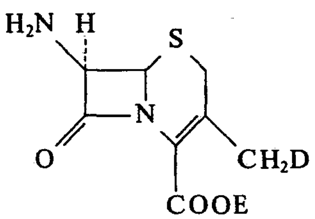

(III)

dans laquelle D et E sont définis comme indiqué plus haut, avec un acide uréidocarboxylique de formule générale IV,

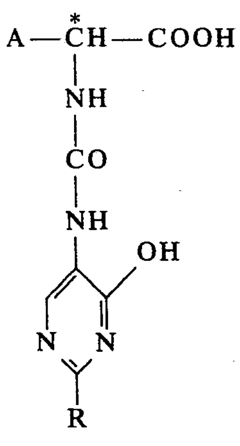

(IV)

dans laquelle A et R possèdent les significations indiquées plus haut, ou avec leurs sels ou dérivés réactifs, entre −40 et +40°C en présence d'un solvant et éventuellement d'une base, ou

b) on fait réagir un dérivé d'acide céphalosporanique de formule générale V ou ses sels avec des bases minérales ou organiques